# EUROPÄISCHE PATENTANMELDUNG

(11) **EP 3 584 010 A1**
(43) Veröffentlichungstag der Anmeldung: **25.12.2019**
(21) Anmeldenummer: 19188161.4
(22) Anmeldetag: 30.07.2012
(51) Int. Cl.: B01L 3/00

(54) **UNTERSUCHUNGSVERFAHREN AN UND/ODER MIT EINEM BIOLOGISCHEN PROBENMATERIAL**

(62) Teilanmeldung aus: 12743441.3
(71) Anmelder: NMI Naturwissenschaftliches und Medizinisches Institut an der Universität Tübingen, 72770 Reutlingen (DE)
(72) Erfinder: STELZLE, Martin, 72766 Reutlingen (DE); SCHÜTTE, Julia, 72072 Tübingen (DE); SIMON, Werner, 72810 Gomaringen (DE); HAGMEYER, Britta, 72070 Tübingen (DE); KUBON, Massimo, 79312 Emmendingen (DE)
(74) Vertreter: Witte, Weller & Partner Patentanwälte mbB

(57) **Zusammenfassung**

Die vorliegende Offenbarung betrifft ein Verfahren zur Durchführung von Untersuchung an und/oder mit biologischem Probenmaterial (35) in einem System, das zumindest einen mikrofluidischen Proben¬anordnungs¬bereich aufweist, in dem biologisches Probenmaterial (35) angeordnet und ggf. kultiviert wird, mit den Schritten: a) das biologische Probenmaterial (35) wird in dem Probenanordnungsbereich (31) assembliert, b) das Probenmaterial (35) wird mit zumindest einer Substanz inkubiert, die über mikrofluidische Kanäle (38) zugeführt wird, und c) das Probenmaterial (35) wird untersucht.

## Beschreibung

Die vorliegende Erfindung betrifft ein System zur Durchführung von Untersuchungen an und/oder mit biologischem Probenmaterial, insbesondere komplexen Zellanordnungen.

Die vorliegende Erfindung betrifft ferner die Verwendung derartiger Systeme bei der Untersuchung von und/oder an biologischem Probenmaterial.

Neben der Untersuchung von natürlich vorkommenden Zellen, die ggf. zu organotypischen Zellkultursystemen assembliert werden, betrifft die vorliegende Erfindung auch die Untersuchung von und/oder mit Tumorzellen und Zelllinien.

In vielen Bereichen der wissenschaftlichen Forschung sowie der Diagnostik, sei es im Forschungslabor oder im Alltag eines mit Routineuntersuchungen befassten Labors, besteht Bedarf an komplexen Zellanordnungen, die unter möglichst physiologischen Bedingungen, also beispielsweise in der anatomisch korrekten Anordnung der einzelnen Zelltypen zueinander vorliegen und/oder physiologisch funktionell perfundiert werden können.

Ein Anwendungsbeispiel für derartige komplexe Zellanordnungen ist die Bestimmung der Toxizität und des Metabolismus von Wirkstoffen in der pharmazeutischen Industrie.

Daher besteht ein Bedarf an komplexen, organotypischen Zellkultursystemen, die aus biologischen Zellen bestehen, die in Umgebungen wachsen, die eine Differenzierung über einen entsprechend langen Zeitraum und/oder eine zur *in vivo-*Situation vergleichbare Funktion ermöglichen.

Von besonderem Interesse ist dabei zum einen eine organotypische Leberzellen-Kokultur, mit der Wirkstoffe auf Toxizität und Metabolisierung getestet werden sollen. Die Leber dient unter anderem dem Abbau und der Ausscheidung von Stoffwechselprodukten, Medikamenten und Giftstoffen, die über das Blutkreislaufsystem in die Leber gelangen. Diese Substanzen werden von den Hepatozyten metabolisiert und über die Gallenflüssigkeit abtransportiert. Die von der Leber produzierte Gallenflüssigkeit gelangt über das Gallengangsystem in den Darm und wird auf diese Wiese ausgeschieden. Für eine organotypische Leberzellkultur für die Medikamententestung ist es wichtig, dass die Hepatozyten nach außen von Endothelzellen besetzt werden, wobei die Perfusion der komplexen Zellkultur von der Seite der Endothelzellen her erfolgt. Die Kokultur der Hepatozyten mit Endothelzellen und gegebenenfalls Sternzellen gewährleistet die gewebetypische Differenzierung der Hepatozyten und damit verbundenen Expression von Genen, die zur Metabolisierung der genannten Stoffe erforderlich sind.

Weiter besteht ein Bedarf an einer organotypischen Gewebestruktur, wie sie beispielsweise im Darm zu finden ist. Auch hier ist es für eine physiologisch funktionelle Perfusion erforderlich, dass zwischen "innen" und "außen" unterschieden wird. Im Darm werden durch den Verzehr aufgenommene Stoffe enzymatisch gespalten und über das Darmepithel in den Blutkreislauf transportiert. Das Darmepithel besteht aus einer einschichtigen Epithelschicht, die dem Darmlumen zugewendet ist, und einer darunterliegenden Schicht von Mesenchymzellen, die die Differenzierung und Funktion der Epithelzellen aufrechterhält. An einem solchen *in vitro* hergestellten Zellverbund könnten Untersuchungen zur Aufnahme von Medikamenten bei oraler Verabreichung durchgeführt werden.

Ein weiteres Anwendungsgebiet ist die sogenannte Blut-Hirn Schranke, die den Übertritt von Substanzen aus dem Blut in das Gehirn kontrolliert und dafür sorgt, dass die chemische Zusammensetzung der Intrazellularflüssigkeiten des Gehirns weitgehend konstant bleibt, was für eine präzise Signalübertragung zwischen den Nervenzellen des Zentralnervensystems notwendig ist. Um Blutgefäße herum wird die Blut-Hirn-Schranke durch Endothelzellen und Astrozyten gebildet. Sie sorgen über aktive Transportsysteme für den Transfer von Nährstoffen und Sauerstoff bzw. Metaboliten. Im Zusammenhang mit der Entwicklung von Wirkstoffen sind Kenntnisse über die Durchlässigkeit der Blut-Hirn-Schranke für diese Wirkstoffe und damit ihre Verfügbarkeit in Bereichen des Nervensystems von besonderem Interesse.

Um diesen Anforderungen gerecht zu werden, beschreibt die DE 10 2008 018 170 B4 ein mikrofluidisches System, das zum Aufbau und zur anschließenden Kultivierung von komplexen Zellanordnungen dient. Das System umfasst mehrere Mikrokanäle, über die es von außen mit einem Medium perfundiert werden kann. Zu diesem Zweck ist das System mit Anschlüssen zur fluidischen Steuerung versehen.

Durch eine spezielle Kanalstruktur mit Öffnungen, an denen inhomogene elektrische Felder angelegt werden, lassen sich in diesem mikrofluidischen System an den Öffnungen komplexe organotypische Zellanordnungen assemblieren.

Das mikrofluidische System ist ferner in unterschiedlichen Bereichen mit unterschiedlichen selektiven Beschichtungen versehen, um die Besiedlung mit den Zellen gezielt zu beeinflussen. Dabei kann die Besiedelung in bestimmten Bereichen durch eine adhäsive Beschichtung unterstützt und in anderen Bereichen durch eine non-adhäsive Beschichtung vermieden werden. Ferner kann eine Beschichtung mit Extrazellulärmatrixproteinen (ECM) vorgesehen sein, um Zellwachstum und -differenzierung zu unterstützen. Wie diese Funktionalisierung der einzelnen Bereiche erfolgt, ist nicht beschrieben.

In diesem System lässt sich bspw. ein organotypisches Lebergewebe erzeugen, bei dem Hepatozyten und Endothelzellen so angesiedelt werden, dass die Hepatocyten anschließend nach außen vollständig von Endothelzellen besetzt sind. Nachdem diese komplexe Struktur aufgebaut wurde, wird sie dann durch zwei Mikrokanäle mit Nährflüssigkeit perfundiert und somit über längere Zeiträume kultiviert. Wenn dem Medium dann Medikamente zugesetzt werden, können diese auf Toxizität und Metabolisierung getestet werden. Dabei ist es von Vorteil, dass die Perfusion der komplexen Zellkultur von der Seite der Endothelzellen her erfolgt, wie es im intakten Lebergewebe der Fall ist.

Im Stand der Technik sind verschiedene Verfahren beschrieben, wie eine ortsaufgelöste Biofunktionalisierung der inneren Oberflächen von mikrofluidischen Systemen realisiert werden kann. Diese Funktionalisierung wird dabei vor der Deckelung, also dem Verschließen des noch offenen Systems durchgeführt, wozu beispielsweise Spotting Verfahren, lithographische Verfahren oder Mikrokontaktprinting verwendet wird.

Derartige Systeme können gemäß der DE 10 2009 039 956 A1 so bereitgestellt werden, dass sie kompatibel sind mit den üblichen Massenfertigungsverfahren, insbesondere Deckelungsverfahren für mikrofluidische Systeme, und dennoch eine ortsaufgelöste, dreidimensionale Biofunktionalisierung ermöglichen.

Dazu wird ein mikrofluidisches System bereitgestellt, bei dem ausgewählte Bereiche der inneren Oberflächen selektiv funktionalisiert sind.

Die Erfinder der DE 10 2009 039 956 A1 konnten zeigen, dass es möglich ist, durch Bestrahlung mit kurzwelligem Licht beliebige Bereiche sowohl der Trägerplatte als auch der Deckelplatte und der Wandbereiche zu funktionalisieren, so dass sich in dem geschlossenen System dann komplexe Zellstrukturen assemblieren lassen, wie dies beispielsweise in der oben erwähnten DE 10 2008 018 170 beschrieben ist.

Dabei ist insbesondere von Vorteil, dass sich so auch Oberflächenbereiche funktionalisieren lassen, die nicht parallel zu der Ebene von Deckelplatte und/oder Trägerplatte liegen, was die spätere Ausbildung komplexer, also dreidimensionaler Zellstrukturen ermöglicht.

Weiter ist von Vorteil, dass die Funktionalisierung schnell und einfach zu erzielen ist, wenn die Eigenschaften der Materialoberflächen von Trägerplatte, Deckelplatte Wandbereichen genutzt werden, um die nicht-funktionalisierten Bereiche ohne weitere Arbeitsschritte bereitzustellen, nur die zu aktivierenden Bereiche müssen dann bestrahlt werden. Dies gilt insbesondere für polymere Materialien, bei denen die UV-Bestrahlung die Bildung von Säuregruppen bewirkt. Andere gängige Materialien für fluidische Mikrosysteme, wie Glas, Silizium oder Siliziumnitrid, müssen zuvor non-adhesiv gemacht werden, was z.B. durch Silanisierung mit einem hydrophoben Silan erreicht werden kann.

Ferner ist von Vorteil, dass die gewählte Funktionalisierung eine lange Lagerzeit der neuen Systeme ermöglicht. Die durch die Bestrahlung erzeugten Säuregruppen sind an sich schon über mehre Monate stabil lagerfähig. Durch Befüllen des permanent geschlossenen Systems mit entsprechenden Gasen oder Flüssigkeiten und nachfolgendes Einschweißen kann aber eine noch erheblich längere Lagerzeit der steril verpackten Systeme gewährleistet werden.

Vor dem Hintergrund dieses Standes der Technik besteht ein anhaltender Bedarf, Mikrofluidiksysteme, die zur Anordnung und Kultivierung einfacher und komplexer Zellanordnungen geeignet sind, mit zusätzlichen Eigenschaften und Funktionen auszustatten, so dass sie nicht nur im Forschungslabor verwendbar sind, sondern auf einfache Weise auch für Massenuntersuchungen und Hochdurchsatzuntersuchungen eingesetzt werden können, wie sie beispielsweise beim Drug Screening Anwendung finden.

Dabei soll die Handhabung derartiger Mikrofluidiksysteme schnell und einfach ermöglicht werden, wobei ein konstruktiv zuverlässiger und preiswerter Aufbau realisiert werden soll.

Unter einem "Mikrofluidiksystem" wird im Rahmen der vorliegenden Erfindung ein System verstanden, das mittels der Mikrosystemtechnologie hergestellt wird. Verschiedene Verfahren, die in der Mikrosystemtechnologie verwendet werden, sind zum Beispiel beschrieben in dem Lehrbuch von W. Menz et al., "Microsystem Technology", WILEY-VCH Verlag GmbH, Weinheim, 2001.

In Mikrosystemen werden beispielsweise mikromechanische, mikrooptische, mikrofluidische und/oder mikroelektronische Komponenten kombiniert. Mikrosysteme werden daher im angelsächsischen Sprachraum auch als MEMS (Micro Electro Mechanical System), BioMEMS (Bio Micro Electro Mechanical Systems) oder MOEMS (Micro Opto Mechanical System), in manchen Publikationen auch als Micromachines bezeichnet.

In vielen Fällen sind in derartigen Mikrosystemen auch Sensoren und/oder Aktoren enthalten.

Die Abmessungen der Mikrosysteme liegen im Mikrometerbereich, Strukturkomponenten auf den Mikrosystemen können Abmaße im Submikrometerbereich aufweisen.

Anwendung finden die Mikrosysteme nicht nur in klassischen Bereichen wie Elektrotechnik, Steuerungstechnik und Informatik, sondern beispielsweise auch in der Medizintechnik, der Biotechnologie und der Pharmakologie. Wenn die Mikrosysteme Kanäle oder Bereiche zur Aufnahme von Flüssigkeiten beinhalten, werden sie als mikrofluidische Systeme oder Mikrofluidiksysteme bezeichnet.

Vor dem Hintergrund dieses Standes der Technik liegt der vorliegenden Anmeldung die Aufgabe zugrunde, ein System der eingangs genannten Art und dessen Elemente bereitzustellen, das bei konstruktiv einfachem und preiswertem Aufbau auch im Routinelabor und für Hochdurchsatzmessungen an Mikrofluidiksystemen geeignet ist. Beispielsweise soll es das System ermöglichen, organtypische Elemente von Leber, Darm, Niere, Haut, Lunge, Herzmuskel, Blutgefäßen, aber auch typische Barrieresystemen wie Blut-Hirn, Darm-Blut, Luft-Lunge aufzubauen.

Erfindungsgemäß wird diese Aufgabe gelöst durch eine Anschlussplatte für ein System zur Durchführung von Untersuchungen an und/oder mit biologischem Probenmaterial, die eine mechanische Aufnahmevorrichtung für einen mikrofluidischen Probenchip, in dem das zu untersuchende biologische Probenmaterial angeordnet und ggf. kultiviert wird, äußere Anschlusselemente, um die Anschlussplatte zur Übertragung von Medien und/oder Signalen lösbar mit einer Kontrolleinheit zu verbinden, innere Anschlusselemente, um die Anschlussplatte zur Übertragung von Medien und/oder Signalen lösbar mit dem Probenchip zu verbinden, sowie vorzugsweise zumindest ein Funktionselement aufweist, das zwischen den äußeren und den inneren Anschlusselementen angeordnet ist.

Ferner wird die der Erfindung zugrunde liegende Aufgabe gelöst durch einen mikrofluidischen Probenchip, der insbesondere für die neue Anschlussplatte vorgesehen ist, mit zumindest einer Probenkanaleinheit, in der ein mikrofluidischer Probenanordnungsbereich vorgesehen ist, in dem biologisches Probenmaterial angeordnet und ggf. kultiviert wird, wobei die Probenkanaleinheit mit einem Anschlussbereich zur Übertragung von Medien und/oder Signalen versehen ist, und vorzugsweise zwischen dem Probenanordnungsbereich und dem Anschlussbereich zumindest ein Funktionselement angeordnet ist.

Ferner wird die der Erfindung zugrunde liegende Aufgabe gelöst durch ein System zur Durchführung von Untersuchungen an und/oder mit in einem Probenanordnungsbereich des neuen Probenchips befindlichem biologischen Probenmaterial, mit der neuen Anschlussplatte zur Aufnahme des Probenchips und mit zumindest einer Kontrolleinheit, mit der die Anschlussplatte zur Übertragung von Medien und/oder Signalen lösbar verbindbar ist.

Die der Erfindung zugrunde liegende Aufgabe wird auf diese Weise vollkommen gelöst.

Die Erfinder der vorliegenden Anmeldung haben nämlich erkannt, dass durch eine hierarchische Anordnung der einzelnen Elemente des neuen Systems der unterschiedlichen Komplexität der einzelnen Elemente und der unterschiedlichen Verwendungshäufigkeit und dem unterschiedlichen Austauscherfordernis Rechnung getragen werden kann.

Durch die lösbaren Verbindungen können dabei Medien und Signale zwischen den einzelnen Elementen des Systems ausgetauscht werden. Zu den Medien zählen Flüssigkeiten, mit denen die Probenchips, insbesondere die Probenaufnahmebereiche gespült und Nährstoffe, Probenmaterial oder Testsubstanzen zugeführt werden, sowie Gase, mit denen die Probenchips, insbesondere die Probenaufnahmebereiche gespült und geflutet werden können. Zu den Signalen zählen elektrische und optische Signale sowie Drucksignale.

Als hierarchisch unterstes Element ist der mikrofluidische Probenchip anzusehen, der reines Verbrauchsmaterial sein kann und nach der Durchführung einer Untersuchung an bestimmtem biologischem Probenmaterial verworfen wird. Ein derartiger Probenchip weist zum Beispiel einen Probenanordnungsbereich auf, wie er in der eingangs erwähnten DE 10 2008 018 170 B4 beschrieben ist, auf deren Inhalt hier vollumfänglich Bezug genommen wird. Wegen prinzipieller Details der Probenaufnahmebereiche wird daher auf die DE 10 2008 018 170 B4 sowie die eingangs ebenfalls erwähnte DE 10 2009 039 956 A1 verwiesen.

Auf dem neuen Probenchip sind erfindungsgemäß ein oder mehrere Probenkanaleinheiten angeordnet, in denen jeweils entsprechende mikrofluidische Probenanordnungsbereiche parallel oder in Serie vorgesehen sind.

Um die Probenkanaleinheit bzw. den darin vorgesehenen Probenanordnungsbereich entsprechend vorzubereiten (zu primen), damit danach darin das biologische Probenmaterial angeordnet, vorzugsweise eine komplexe Zellanordnung etabliert werden kann, ist der Probenchip mit einem Anschlussbereich versehen, über den er zur Übertragung von Medien und Signalen lösbar kontaktiert werden kann.

Auf diese Weise ist es möglich, zunächst eine Lösung zur Aktivierung funktionalisierter Bereiche in die Probenkanaleinheit einzuleiten, danach ggf. Spüllösungen hindurchzuleiten, um schließlich die so aktivierten Bereiche mit entsprechendem biologischem Material zu belegen.

Durch fluidische Anschlüsse kann dazu Medium mit biologischem Probenmaterial durch die Probenkanaleinheit geleitet werden, um das biologische Probenmaterial in den Probenkanaleinheiten anzusammeln.

Über elektrische Kontaktierungen können dabei elektrische Felder angelegt werden, die gemäß dem zuvor beschriebenen Stand der Technik dazu führen, dass sich komplexe Zellanordnungen auch aus unterschiedlichen Zellarten und -typen in dem Probenanordnungsbereich etablieren, so dass sie dort kultiviert werden können.

Zwischen dem Probenanordnungsbereich und dem Anschlussbereich ist bevorzugt zumindest ein Funktionselement angeordnet, über das beispielsweise elektrische Messsignale oder flüssige Messproben von dem kultivierten Probenmaterial genommen werden können. Als Funktionselemente können dazu Sensoren und/oder Pumpen in den Probenchip integriert werden.

Diese Funktionselemente können auch der Begasung der biologischen Probe sowie der fluidischen Steuerung dienen, um sicherzustellen, dass sämtliche Probenkanaleinheiten auf dem Probenchip zuverlässig mit Medium gefüllt sind. Dazu können Blasenfallen (bubble traps) in den Probenchip integriert werden, um zu verhindern, dass die Zellanordnungen durch Luftblasen gestört oder verändert werden.

Sozusagen die Verbindung zu der Außenwelt stellt die erfindungsgemäß vorgesehene Anschlussplatte bereit, auf der eine mechanische Aufnahmevorrichtung vorgesehen ist, in die ein mikrofluidischer Probenchip eingesetzt werden kann. Der eingesetzte Probenchip ist dann über seinen Anschlussbereich lösbar mit den inneren Anschlusselementen der Anschlussplatte kontaktiert, so dass über die äußeren Anschlusselemente der Anschlussplatte die erforderlichen Medien und Signale zugeführt werden können und flüssiges Verbrauchsmaterial abgeführt werden kann. Ferner können über die äußeren Anschlusselemente Messsignale und flüssige Messproben von den jeweiligen Probenanordnungsbereichen entnommen werden.

Auch auf der Anschlussplatte ist vorzugsweise zumindest ein Funktionselement vorgesehen, das zwischen den äußeren und den inneren Anschlusselementen angeordnet ist und dazu dient, beispielsweise die zugeführten Flüssigkeiten gleichmäßig oder gesteuert zu verteilen und die zugeführten und abgeleiteten elektrischen Signale entsprechend zu steuern und zuzuordnen.

Unter einem Funktionselement wird im Rahmen der vorliegenden Erfindung folglich ein Element verstanden, das auf der Anschlussplatte zwischen den äußeren und inneren Anschlusselementen bzw. auf dem Probenchip zwischen dem Probenanordnungsbereich und dem Anschlussbereich sowie ggf. auch in dem Probenanordnungsbereich selbst angeordnet ist. Ein Funktionselement wirkt in einer Ausgestaltung aktiv oder passiv auf Medien und/oder Signale, die zwischen den äußeren und inneren Anschlusselementen bzw. zwischen dem Probenanordnungsbereich und dem Anschlussbereich ausgetauscht werden. In einer weiteren Ausgestaltung können Funktionselemente als Sensoren ausgebildet sein, die der Erfassung von Messwerten dienen.

Während der mikrofluidische Probenchip nicht in jedem Fall wiederverwendbar ist, ist die Anschlussplatte nach Durchführung einer entsprechenden Untersuchung an einem eingesetzten Probenchip wiederverwendbar.

Die Anschlussplatte ist zwar notwendiger Bestandteil des übergeordneten Systems, aber auch selbst ein Austauschelement, so dass für unterschiedliche Anwendungen, Anforderungen und Probenchips verschiedene Anschlussplatten vorgesehen sein können, die jedoch jeweils in dem System mit der vorgesehenen Kontrolleinheit verwendet werden.

Diese Kontrolleinheit ist lösbar mit der Anschlussplatte verbunden, so dass sie nacheinander mit unterschiedlichen Anschlussplatten verbunden werden kann.

Die Kontrolleinheit dient dazu, die Zufuhr der Medien sowie die Zuleitung der Signale zu steuern und den Abfluss des Verbrauchsmaterials sowie die Entnahme von flüssigen Messproben und Messsignalen zu steuern und ggf. für deren Speicherung zu sorgen.

Besonders bevorzugt ist es dabei, wenn zwei unterschiedliche Kontrolleinheiten vorgesehen sind, einmal eine Dielektrophoreseeinheit, die zum Primen des Probenchips und zum Assemblieren des Probenmaterials in dem Probenanordnungsbereich dient, und zum anderen eine Perfusionseinheit, die nach der Anordnung des Probenmaterials zum Einsatz kommt.

Die Dielektrophoreseeinheit kann auch dazu verwendet werden, um nacheinander verschiedene Zelltypen in den Probenanordnungsbereich zu leiten, so dass dort organotypische Zellanordnungen entstehen, wie es in der eingangs erwähnten DE 10 2008 018 170 B4 prinzipiell für den Laborbetrieb beschrieben ist.

Sobald eine entsprechende Anschlussplatte bzw. der auf der Anschlussplatte vorgesehene Probenchip entsprechend mit einer zu untersuchenden biologischen Probe versehen ist, wird die Dielektrophoreseeinheit von der Anschlussplatte abgetrennt und jetzt die als Kontrolleinheit verwendete Perfusionseinheit angeschlossen. Die Perfusionseinheit dient vor allem der Versorgung des Probenmaterials mit Nährmedium und der Steuerung der Inkubation des Probenmaterials über einen bestimmten Messzeitraum sowie zur Erfassung von elektrischen Messwerten und/oder zur Entnahme von flüssigen Messproben. Ferner können über die Perfusionseinheit Testsubstanzen zugeführt werden.

Beide Kontrolleinheiten können in einem Gerät realisiert sein, wobei es vorteilhaft ist, wenn sie als getrennte Geräte ausgebildet sind.

Die Dielektrophoreseeinheit ist nämlich in der Regel die komplexer aufgebaute Kontrolleinheit, sie kann nach der Etablierung einer komplexen Zellanordnung in einem Probenchip erneut dafür verwendet werden, in einem anderen Probenchip eine Zellanordnung zu etablieren, während der zuvor so vorbereitete Probenchip, der sich nach wie vor in "seiner" Anschlussplatte befindet, über die Perfusionseinheit im Dauerbetrieb während des Messzeitraumes versorgt, gesteuert und messtechnisch erfasst wird.

Somit ist nur eine komplexe Kontrolleinheit zur Etablierung der Zellanordnung erforderlich, nämlich die Dielektrophoreseeinheit, während mehrere einfacher und damit preiswerter aufgebaute Perfusionseinheiten vorgesehen sind, um die jeweils vorbereiteten Probenchips in ihren Anschlussplatten zu versorgen und Messwerte und - proben zu nehmen.

Dabei ist es bevorzugt, wenn das zumindest eine Funktionselement in der Anschlussplatte und/oder dem Probenchip dazu ausgelegt ist, zumindest eine Aufgabe zu erfüllen, die ausgewählt ist aus der Gruppe, die die elektrische und optische Aufbereitung, Verteilung und Zusammenführung sowie Zwischenspeicherung von Steuer- und Messsignalen, die Speicherung, den Transport und die Verteilung von Flüssigkeiten und Gasen, das Abtrennen von Gasbestandteilen aus den Flüssigkeiten in aktiven oder passiven Blasenfallen, die Erzeugung elektrischer Pulse, die Erzeugung elektrischer und/oder magnetischer Gleich- und/oder Wechselfelder, sowie die Übertragung von Drucksignalen umfasst.

Das zumindest eine Funktionselement kann beispielsweise dazu ausgebildet sein, zur Zelllyse elektrische Pulse an in dem Probenanordnung befindliche Zellen anzulegen, um diese Zellen - ggf. unter Zugabe von Lysereagentien - aufzuschließen und deren Inhalt im Effluent untersuchen zu können. Ferner kann es der Erzeugung magnetischer Felder dienen, die für den Transport von Flüssigkeiten, beispielsweise als Pumpen, eingesetzt werden können.

Dabei ist es bevorzugt, wenn die inneren Anschlusselemente der Anschlussplatte zum blasenfreien Ankoppeln an den Probenchip und die äußeren Anschlusselemente zum blasenfreien Ankoppeln an die Kontrolleinheit ausgebildet sind.

Hier ist von Vorteil, dass beim Einsetzen und Anschließen des Probenchips keine störenden Luftblasen in die Probenanordnungsbereiche und beim An- und Abkoppeln der Anschlussplatte an der Kontrolleinheit keine störenden Luftblasen in die Mikrokanäle der Anschlussplatte gelangen, die sich dort störend auswirken würden.

Weiter ist es bevorzugt, wenn die Aufnahmevorrichtung Spannelemente zum mechanischen Halten des Probenchips aufweist, vorzugsweise in der Aufnahmevorrichtung eine optisch dünne Abdeckung vorgesehen ist.

Mit diesen Merkmalen ist der Vorteil verbunden, dass der Probenchip durch einfache Maßnahmen für Auflicht- oder Durchlichtmikroskopie zugänglich wird.

Bei dem neuen Probenchip und der neuen Anschlussplatte ist es bevorzugt, wenn das zumindest eine Funktionselement als optisch empfindlicher, elektrischer, chemischer oder biochemischer Sensor ausgelegt ist.

Hier ist von Vorteil, dass eine *in-situ* Qualitätsüberwachung der durchgeführten Experimente sowie die online Erfassung von Messgrößen möglich ist.

Unter einem optisch empfindlichen Sensor wird im Rahmen der vorliegenden Erfindung sowohl ein optoelektronischer Sensor als auch ein Sensor verstanden, der auf rein optisch wirkenden Verstärkungseffekten beruht.

Ferner ist es bevorzugt, wenn in dem Probenanordnungsbereich zumindest zwei Elektroden zur Erzeugung eines elektrischen Feldes vorgesehen sind, wobei die Elektroden vorzugsweise zur Impedanzmessung, weiter vorzugsweise zur Erzeugung elektrischer Pulse ausgelegt sind.

Hier ist von Vorteil, dass die Elektroden mehreren Zwecken dienen können, was den mechanischen Aufbau des Probenchips vereinfacht.

Dabei ist es bevorzugt, wenn die Elektroden als elektrisch leitende Polymerelektroden ausgebildet sind.

Auch diese Maßnahme ist konstruktiv von Vorteil, denn die Polymerelektroden lassen sich einfacher in den Probenchip integrieren als Metallelektroden. Dazu ist es lediglich erforderlich, eine Lösung mit zumindest einem leitfähigen, polymerisierbaren Monomer in entsprechende Mikrokanäle einzufüllen, die zu für die Ausbildung von Anschlussstellen und Elektroden vorgesehenen Bereichen führen, und die Lösung aushärten zu lassen. Das Aushärten kann durch Wärmebehandlung, Bestrahlung mit UV-Licht oder andere geeignete Maßnahmen erfolgen.

Polymerelektroden lassen sich beispielsweise mit dotierten Epoxidharzen, oder Silikonen (z.B. PDMS) herstellen. Dafür wird ein leitfähiges Dotiermittel, wie zum Beispiel Graphen, Kohlenstoffnanoröhrchen oder Metallnanopartikel, mit dem Monomer und einem geeigneten Vernetzer vermischt und die pastöse Lösung in die Mikrokanäle gefüllt. Die Aushärtung erfolgt dann z.B. über Temperaturerhöhung.

Polymerelektroden lassen sich auch mit photovernetzbaren Polyelektrolyten herstellen, z.B. mit Polydiallyldimethylammoniumchloride (PDADMAC) oder Poly-2-acrylamino-2-methyl-1-propanesulfonsäure (PAMPSA).

Weiter ist es bevorzugt, wenn in dem Probenanordnungsbereich zumindest ein von zwei Stegen begrenzter Spalt vorgesehen ist, der zwei mikrofluidische Kanäle miteinander verbindet, und in dem eine Trennstruktur angeordnet ist, die vorzugsweise eine Membran mit Löchern oder ein Hydrogel umfasst.

Schließlich ist es bevorzugt, wenn der Probenchip ein Trägersubstrat umfasst, in dem die zumindest eine Probenkanaleinheit ausgebildet ist, und das mit einem Deckel verschlossen ist, der vorzugsweise zumindest bereichsweise optisch dünn ausgelegt ist.

Hier ist von Vorteil, dass das Probenmaterial in dem Probenanordnungsbereich mit optischen Methoden untersucht werden kann.

Allgemein ist es noch bevorzugt, wenn der Probenchip zumindest eine passive Blasenfalle mit einer Membran umfasst.

Die mikrofluidische Struktur ist dabei so ausgelegt, dass Luftblasen zu der Membran wandern, über die sie aus dem mikrofluidischen System austreten können, weil die Membran für Gase permeabel ist.

Mit dem neuen System lassen sich wegen der erfindungsgemäß vorgesehenen Systemhierarchie und -modularität sowie der erfindungsgemäß vorgesehenen Eigenschaften und Merkmale erstmals viele Anwendungen und Assays entweder überhaupt oder zumindest mit bisher nicht erreichbarer Effizienz und Präzision durchführen.

Derartige, mit dem neuen System und seinen Elementen durchführbare Verfahren zur Untersuchung an und/oder mit biologischem Probenmaterial umfassen die Schritte:
a) das biologische Probenmaterial wird in dem Probenanordnungsbereich assembliert,
b) das Probenmaterial wird mit zumindest einer Substanz inkubiert, die über mikrofluidische Kanäle zugeführt wird, und
c) das Probenmaterial wird untersucht.

Das neue Verfahren ermöglicht die reproduzierbare Analyse von geringen Mengen an Probenmaterial unter kontrollierten Bedingungen mit quasikontinuierlicher Probenentnahme über längere Zeiträume. Es ist daher nicht nur mit dem neuen System und seinen Einheiten sondern auch generell mit einem System durchführbar, das zumindest einen mikrofluidischen Probenanordnungsbereich aufweist, in dem biologisches Probenmaterial angeordnet und ggf. kultiviert werden kann.

Unter einer Substanz wird im Rahmen der vorliegenden Erfindung sowohl ein Wirkstoff, zum Beispiel in Form einer monomeren oder polymeren Verbindung oder in Form von Partikeln, verstanden, dessen qualitative und/oder quantitative Wirkung auf das Probenmaterial untersucht werden soll, als auch weiteres biologisches Material, dessen Wechselwirkung mit dem Probenmaterial untersucht werden soll. Pharmazeutische Wirkstoffe und potentiell toxische Stoffe fallen ebenso unter dieses Verständnis wie beispielsweise Tumorzellen, deren Invasion in das Probenmaterial untersucht werden soll.

Ferner fallen auch Nährstoffe, Kulturmedien oder andere Stoffe unter dieses Verständnis, die beispielsweise zum Anfärben, Aufschließen oder zur sonstigen chemischen, biologischen, biochemischen oder physikalischen Beeinflussung des Probenmaterials oder dessen Nährstoffversorgung dienen.

In einer Weiterbildung ist es bevorzugt, wenn in Schritt a) zumindest ein organähnliches Zellkulturmodell in dem Probenanordnungsbereich etabliert wird.

Hier ist von Vorteil, dass eine Untersuchung an Zellkulturmodellen in einer *in-vivo* ähnlichen Situation möglich wird, wobei dennoch eine fortlaufende Beobachtung und Probengewinnung realisiert werden kann. Die Konzentration von Zellprodukten kann direkt im Perfundat untersucht werden.

Weiter ist es bevorzugt, wenn in Schritt c) mit zumindest einem Analyseverfahren eine Eigenschaft oder Eigenschaftsänderung des Probenmaterials untersucht wird, die ausgewählt ist aus Metabolom, Enzymaktivität, Proteom, Genom, Genexpression, Sekretion von Markern, morphologische Erscheinung, Zellvitalität, Zellorganellenfunktion, Interaktion zwischen Zellen, Interaktion zwischen Zellen und Umgebung im Probenanordnungsbereich, Proliferation.

Im Schritt b) werden die Zellen dazu bspw. durch Zugabe von geeigneten Substanzen lysiert, und in Schritt c) die DNA, RNA oder Proteine extrahiert und analysiert. Diese Schritte dienen der schnellen Untersuchung von Änderungen der Zellkultur, was den Vorteil hat, dass der Abbau der DNA, RNA, Proteine im Vergleich mit üblichen Verfahren minimiert wird.

In Schritt c) können ferner strukturelle Veränderungen von Proteinen untersucht werden, beispielsweise eine andere Faltung mit einhergehender Veränderung oder gar Verlust der Funktionalität. Ferner lassen sich Ausbildung oder Verlust von Zell-Zell-Kontakten beispielsweise bei der Blut-Hirn-Schranke (z.B. Verlust von tight junctions) untersuchen. Ferner kann beobachtet werden, wie sich Zellen auf den Oberflächen im Probenanordnungsbereich verhalten, ob sie sich zum Beispiel ausbreiten oder ablösen.

Ferner ist es bevorzugt, wenn in Schritt c) die Zellen elektrisch über in dem Probenanordnungsbereich integrierte Elektroden aufgeschlossen werden.

Hier ist von Vorteil, dass ein Verzicht auf lysierende Reagenzien möglich ist, was eine Verunreinigung und Beeinflussung der Auswertung vermeidet. Besonders vorteilhaft ist dabei, dass die für die Assemblierung vorgesehenen Elektroden auch für den elektrischen Aufschluss verwendet werden können, indem über sie elektrische Pulse geeigneter Stärke und Dauer abgegeben werden.

Dabei ist es bevorzugt, wenn das Analyseverfahren ausgewählt ist aus Immunhistochemie, Mikroskopie, Elektronenmikroskopie, Anfärbung von Zellbestandteilen, Analyse von gelösten Bestandteilen mittels Trennverfahren (Chromatographie, Elektrophorese, Massenspektrometrie), Colorimetrie, Immunoassays, Nukleinsäure-Analytik, PCR (Polymerase Chain Reaction), FISH (Fluorescence in situ hybridization), DNA-Sequenzierung, Spektroskopieverfahren (Infrarotspektroskopie, Ramanspektroskopie, NMR-Spektroskopie, Fluoreszenz-, UV/VIS-Spektroskopie).

Zur Analyse von gelösten Bestandteilen wird beispielsweise das Perfundat untersucht.

Die Anfärbung von Zellbestandteilen umfasst beispielsweise die Interaktion von markierten Antikörpern mit Proteinen, die Kernfärbung und die Färbung des Cytosols (beispielsweise mit Calcein).

Ferner ist es bevorzugt, wenn in Schritt c) die qualitative und/oder quantitative Wirkung der zugeführten zumindest einen Substanz auf das Probenmaterial untersucht wird.

Dieser Schritt ermöglicht die Bestimmung der Toxizität und/oder Wirksamkeit von Substanzen und die Bestimmung von Dosis-Zeitprofilen. Durch wiederholte Inkubation mit Erholungsphasen kann zudem die Langzeitwirkung von Wirkstoffen untersucht werden.

Besonders bevorzugt ist es, wenn eine vergleichende Untersuchung an einem Probenmaterial, das mit der Substanz inkubiert wird, und einem Probenmaterial, das nicht oder mit einer anderen Substanz inkubiert wird, durchgeführt wird.

Hier ist von Vorteil, dass eine Vergleichsmessung an zwei identischen Zellkulturen, einmal behandelt, einmal unbehandelt, möglich wird. Wegen der sehr genau kontrollierten Parameter lassen sich mit den oben beschriebenen Vorteilen des neuen Verfahrens so bisher nicht erzielbare Aussagen über die Wirkung von Substanzen erzielen. Dieser Schritt kann auch zur vorherigen Kalibrierung des Systems verwendet werden.

Allgemein ist es bevorzugt, wenn in Schritt a) ein leberähnliches Zellkulturmodell assembliert und kultiviert wird, das in Schritt b) mit zumindest einer potentiell toxischen Substanz inkubiert wird, wobei in Schritt c) die Wirkung der Substanz auf zumindest eine Zellfunktion untersucht wird.

Zu den zu untersuchenden Zellfunktionen der leberähnlichen Zellkultur zählen insbesondere Mitochondriale Dysfunktionen, Induktion oder Inhibition von Cytochrome P450 Enzymen, Beeinträchtigung der Transporterproteinfunktion, Akkumulation von reaktiven Sauerstoffspezies, Beeinträchtigung der Zell-Zell-Interaktionen, Immunreaktionen.

Ferner ist es bevorzugt, wenn in Schritt a) Tumorzellen in einer organähnlichen Zellkultur assembliert und kultiviert werden, die in Schritt b) mit zumindest einem onkologischen Wirkstoff inkubiert wird, wobei in Schritt c) eine Veränderung der Zellkultur untersucht wird.

Hier ist von Vorteil, dass eine Wirkstofftestung an onkologischem Material möglich wird, die unter reproduzierbaren und kontrollierten Bedingungen mit den oben beschriebenen Vorteilen des neuen Verfahrens abläuft. Dabei können ein oder mehrere Wirkstoffe zeitlich nacheinander oder zeitgleich appliziert werden. Tumorwachstum oder Tumorschrumpfen kann beobachtet werden.

Andererseits ist es bevorzugt, wenn in Schritt a) eine biologische Zellbarriere in einer organähnlichen Zellkultur assembliert und kultiviert wird, die in Schritt b) auf einer Seite der Zellbarriere mit zumindest einem Wirkstoff inkubiert wird, wobei in Schritt c) die Konzentration des Wirkstoffes auf der anderen Seite der Zellkultur untersucht wird.

Hier ist von Vorteil, dass mit den oben beschriebenen Vorteilen des neuen Verfahrens die Barrieregängigkeit von Wirkstoffen und der Einfluss von Wirkstoffen auf die Funktion der Barriere untersucht werden können. Beispiele für Barrieren sind Blut-Hirn, Darm-Blut, Blut-Harn, Luft-Blut.

Schließlich ist es bevorzugt, wenn in Schritt a) biologische Zellen in einer organähnlichen Zellkultur assembliert und kultiviert werden, die in Schritt b) mit zumindest einem Pathogen inkubiert wird, wobei in Schritt c) die Reaktion der Zellkultur auf die Inkubation mit dem zumindest einen Pathogen untersucht wird.

Hier sind Messungen der Interaktion von Pathogenen mit Gewebe auf reproduzierbare Weise und mit den oben beschriebenen Vorteilen des neuen Verfahrens möglich. Als Pathogene werden Bakterien, Viren, Parasiten eingesetzt.

Andererseits ist es bevorzugt, wenn in Schritt a) biologische Zellen in einer organähnlichen Zellkultur assembliert und fixiert werden, an die in Schritt b) zumindest ein Färbemittel gebunden wird, wobei in Schritt c) die Färbung mittels optischer Verfahren untersucht wird.

Das Färbemittel kann selbst ein spezifisch an Strukturen der fixierten Zellen bindendes Molekül sein, oder es wird ein spezifisch an Strukturen der fixierten Zellen bindendes Molekül verwendet, an das in einem zweiten Schritt das Färbemittel gebunden wird. Dieser Schritt ermöglicht eine immunhistochemische Anfärbung von organähnlichen Zellkulturen.

Weiter ist es bevorzugt, wenn in Schritt a) biologische Zellen in einer organähnlichen Zellkultur assembliert und fixiert werden, die in Schritt b) getrocknet sowie mit einem Kontrastmittel inkubiert und dann in eine Matrix eingebettet werden, wobei in Schritt c) die in die Matrix eingebetteten Zellen entnommen und untersucht werden.

Mit den oben beschriebenen Vorteilen des neuen Verfahrens können so Zellen beispielsweise für die Elektronenmikroskopie präpariert werden.

Ferner ist es bevorzugt, wenn in Schritt a) biologische Zellen in einer organähnlichen Zellkultur assembliert und kultiviert werden, die in Schritt b) mit Tumorzellen inkubiert wird, wobei in Schritt c) das Verhalten der Tumorzellen und der Zellkultur untersucht wird.

Diese Schritte ermöglichen die Untersuchung der Metastasenbildung und der Verdrängung der gesunden Zellen. Mit den oben beschriebenen Vorteilen des neuen Verfahrens ist erstmals eine kontinuierliche Untersuchung der Tumorentstehung an demselben Gewebe zu verschiedenen Zeitpunkten möglich.

Die Interaktion der Tumorzellen mit der Zellkultur wird dabei vorzugsweise durch Dielektrophorese unterstützt oder gar erst bewirkt.

Es ist weiter bevorzugt, wenn in Schritt a) biologische Zellen in einer organähnlichen Zellkultur assembliert und kultiviert werden, die in Schritt b) mit zumindest zwei Wirkstoffen inkubiert wird, wobei in Schritt c) das Verhalten der Zellkultur untersucht wird.

Hier ist von Vorteil, dass eine sequentielle oder parallele Verabreichung von Wirkstoffen möglich ist, so dass mit den oben beschriebenen Vorteilen des neuen Verfahrens die gegenseitige Beeinflussung der Wirkstoffe untersucht werden kann.

Allgemein ist es noch bevorzugt, wenn in Schritt a) in einem ersten Probenanordnungsbereich biologische Zellen in einer ersten organähnlichen Zellkultur und in einem zweiten Probenanordnungsbereich biologische Zellen in einer zweiten organähnlichen Zellkultur assembliert und kultiviert werden, wobei vorzugsweise der erste und der zweite Probenanordnungsbereich fluidisch in Reihe geschaltet sind, wobei in Schritt b) die erste Zellkultur mit zumindest einem Wirkstoff perfundiert wird, und die zweite Zellkultur mit dem Effluent der ersten Zellkultur perfundiert wird, und wobei in Schritt c) die Reaktion der zweiten Zellkultur auf die Perfusion mit dem Effluent untersucht wird.

Gemäß dieser Weiterbildung können zwei oder mehr auch unterschiedliche organähnliche Strukturen auf einem Probenchip assembliert und untersucht werden, wobei die verschiedenen Probenanordnungsbereiche auf dem Probenchip unterschiedlich ausgebildet sein und selektiv elektrisch angesprochen werden können. Dies ist beispielsweise für die Etablierung einer in-vivo ähnlichen Nierenstruktur wichtig.

Diese Ausgestaltung des neuen Verfahrens ermöglicht auch erstmals die reproduzierbare Untersuchung der Reaktion eines nachgeordneten Organs auf die Behandlung eines anderen Organs mit Wirkstoffen. Dabei wird beispielsweise das Produkt einer ersten Zellkultur in ihrer Wirkung auf nachfolgende Zellkulturen untersucht.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der beigefügten Zeichnung dargestellt und werden in der nachfolgenden Beschreibung näher erläutert. Es zeigen:
- Fig. 1: eine schematische Darstellung des neuen Systems mit Kontrolleinheit und Anschlussplatte;
- Fig. 2: den prinzipiellen, nicht maßstabsgetreuen Aufbau eines Probenchips, wie er in die Anschlussplatte aus Fig. 1 eingesetzt werden kann;
- Fig. 3: den prinzipiellen, nicht maßstabsgetreuen Aufbau einer Probenkanaleinheit, wie sie auf dem Probenchip aus Fig. 2 vorgesehen ist;
- Fig. 4: nicht maßstabsgetreue Ausführungsbeispiele für Probenkanaleinheiten gemäß Fig. 3;
- Fig. 5: ein nicht maßstabsgetreues Ausführungsbeispiel für einen Probenanordnungsbereich aus der Probenkanaleinheit aus Fig. 4;
- Fig. 6: in einer perspektivischen Darstellung ein konkretes Ausführungsbeispiel für die Anschlussplatte aus Fig. 1;
- Fig. 7: einen Längsschnitt durch einen Fluidikblock der Anschlussplatte aus Fig. 6;
- Fig. 8: ein prinzipielles Ausführungsbeispiel für ein erstes Funktionselement, das der Erzeugung eines elektrischen Feldes in dem Probenanordnungsbereich aus Fig. 5 dient;
- Fig. 9: eine perspektivische Darstellung eines Ausschnitts aus einem in Spritzgusstechnik hergestellten Probenchip;
- Fig. 10: verschiedene prinzipielle Ausführungsbeispiele für Aufteilungs- und Kombinationsstellen, wie sie in der Probenkanaleinheit aus Fig. 4 verwendet werden;
- Fig. 11: Ausführungsbeispiele für Funktionselemente, die der Ankopplung und Entgasung der Mikrofluidikkanäle in der Probenkanaleinheit aus Fig. 4 dienen;
- Fig. 12: ein Ausführungsbeispiel zur blasenfreien Ankopplung der Anschlussplatte an die Kontrolleinheit;
- Fig. 13: Ausführungsbeispiele zur Ansammlung von komplexen Zellanordnungen in einem Probenanordnungsbereich mit einer als Hydrogel ausgebildeten Trennstuktur;
- Fig. 14: ein Ausführungsbeispiel zur Ansammlung von komplexen Zellanordnungen in einem Probenanordnungsbereich mit einer als Membran ausgebildeten Trennstruktur; und
- Fig. 15: Diagramme, die die Erfassung von im Probenchip durch verschiedene Zellen gebildetem a) Albumin und b) 6β-Hydroxytestosteron aus Testosteron zeigen.

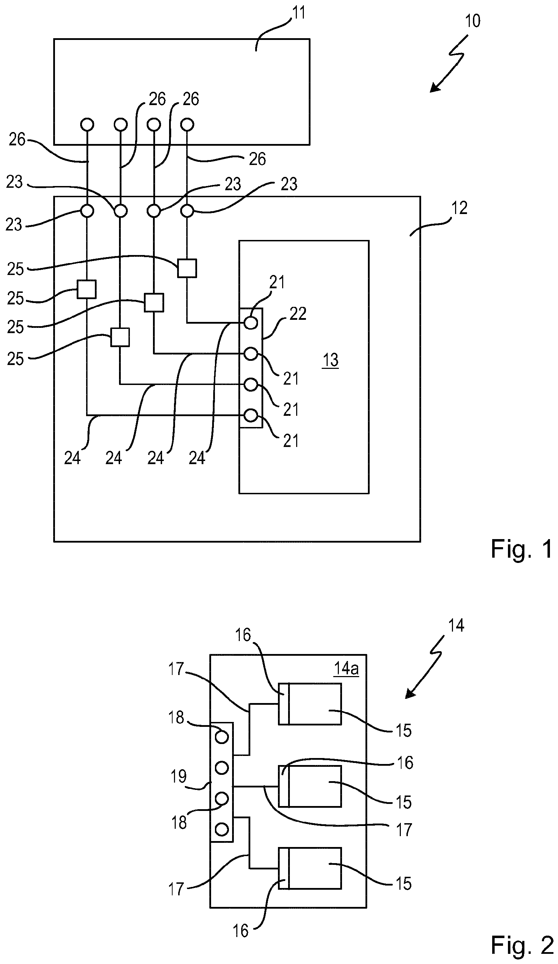

In Fig. 1 ist mit 10 allgemein ein schematisch dargestelltes System zur Durchführung von Untersuchungen an biologischem Probenmaterial bezeichnet, das aus verschiedenen Elementen besteht.

Das System 10 umfasst als erstes Element eine Kontrolleinheit 11, mit der wahlweise als zweite Elemente verschiedene Anschlussplatten 12 verbindbar sind, an die als dritte Elemente über je eine mechanische Aufnahmevorrichtung 13 verschiedene mikrofluidische Probenchips 14 angekoppelt werden können, für die in Fig. 2 ein Ausführungsbeispiel gezeigt ist.

Die Aufnahmevorrichtung 13 dient der betriebssicheren Verankerung der Probenchips 14 und ihrer Kontaktierung. Ferner ermöglicht sie eine optische Analyse von biologischem Probenmaterial, das auf den Probenchips 14 assembliert, kultiviert, inkubiert und perfundiert werden kann. Dazu ist eine optische Analyse mittels Durchlichtmikroskopie oder zumindest mittels Auflichtmikroskopie möglich.

Der Probenchip 14 aus Fig. 2 umfasst ein Trägersubstrat 14a, auf dem beispielhaft drei mikrofluidische Probenkanaleinheiten 15 ausgebildet sind, die jeweils einen eigenen Anschlussbereich 16 für Medien sowie Signale aufweisen. Im einfachsten Fall enthält jeder Probenchip 14 eine Probenkanaleinheit 15.

Jeder Anschlussbereich 16 ist insbesondere über Anschlussleitungen 17 für Medien und Signale mit Chip-Anschlusselementen 18 verbunden, die in dem gezeigten Ausführungsbeispiel in einem linearen Array 19 angeordnet sind.

Die Anschlusselemente 18 wirken mit inneren Anschlusselementen 21 der Anschlussplatte 12 zusammen, die an dem mechanischen Aufnahmebereich 13 der in Fig. 1 gezeigten Anschlussplatte 12 vorgesehen sind. Auch die inneren Anschlusselemente 21 sind hier als lineares Array 22 angeordnet.

Die Anschlusselemente 18 dienen insbesondere auch dem blasenfreien Anschluss des Probenchips 14 an die Anschlussplatte 12.

Die inneren Anschlusselemente 21 sind mit äußeren Anschlusselementen 23 der Anschlussplatte 12 über Anschlussleitungen 24 für Medien und Signale verbunden.

Die äußeren Anschlusselemente 23 dienen insbesondere auch dem blasenfreien Ankoppeln der Anschlussplatte 12 an die Kontrolleinheit 11.

Zwischen den äußeren Anschlusselemente 23 und den inneren Anschlusselemente 21 sind schematisch angedeutete Funktionselemente 25 vorgesehen, die in noch zu beschreibender Weise der Beeinflussung der Medien sowie der Signale dienen, die zwischen den Probenkanaleinheiten 15 und den äußeren Anschlusselementen 23 ausgetauscht werden. Funktionselemente 25 können auch zur Erfassung von Messsignalen ausgebildet sein.

Die äußeren Anschlusselemente 23 sind über weitere Anschlussleitungen 26 für Medien und Signale lösbar mit der Kontrolleinheit 11 verbunden.

Wie bereits eingangs erwähnt, ist die Kontrolleinheit 11 z.B. eine Dielektrophoreseeinheit, die dazu dient, in den Probenkanaleinheiten 15 biologisches Probenmaterial, insbesondere organotypische Zellanordnungen, anzuordnen, wie dies prinzipiell für den Laborbereich aus der DE 10 2008 018 170 B4 bekannt ist.

Nachdem das Probenmaterial entsprechend in den Probenkanaleinheiten 15 angeordnet wurde, kann die Dielektrophoreseeinheit gegen eine Perfusionseinheit ausgetauscht werden, die jetzt als Kontrolleinheit 11 dient und während der Inkubation des Probenmaterials in den Probenkanaleinheiten 15 der Versorgung des Probenmaterials mit Medium sowie der Zufuhr von Testsubstanzen dient. Ferner werden über die Perfusionseinheit Messsignale und flüssige Messproben entnommen.

Die Funktionselemente 25 dienen auch der Prozessierung und Weiterleitung von Medien und Signalen, die zwischen der Kontrolleinheit 11 und den Anschlussbereichen 16 auf den Probenchips 14 ausgetauscht werden. Dazu zählen bspw. die elektrische und optische Aufbereitung, Verteilung und Zusammenführung sowie Zwischenspeicherung von Steuer- und Messsignalen, die Speicherung, der Transport und die Verteilung von Flüssigkeiten und Gasen, das Abtrennen von Gasbestandteilen aus den Flüssigkeiten in aktiven oder passiven Blasenfallen, die Übertragung von Drucksignalen.

Die Funktionselemente 25 können dazu als Ventile, Multiplexer, Pumpen, Heizungen, Kühlungen ausgebildet sein,

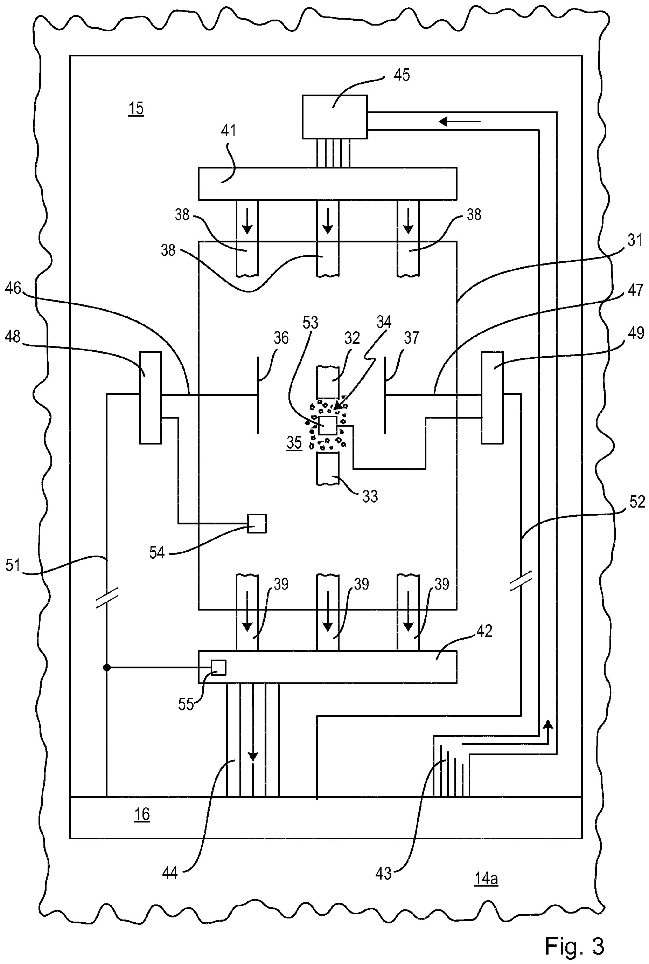

Der prinzipielle Aufbau einer auf dem Trägersubstrat 14a ausgebildeten Probenkanaleinheit 15 ist in Fig. 3 schematisch, vereinfacht und nicht maßstabsgetreu dargestellt.

Das Trägersubstrat 14a besteht ganz oder zumindest im Bereich eines mikrofluidischen Probenanordnungsbereiches 31 innerhalb der Probenkanaleinheit 15 aus optisch dünnem Material, bspw. aus Polycarbonat (PC), Cycloolefincopolymer (COC), Polymethylmethacrylat (PMMA), Polydimethylsolixan (PDMS), oder aus Glas bzw. reinem Silizium. Das Trägersubstrat 14 kann dabei hergestellt sein durch Abformen, Umformen, lithographische Prozesse, Hinzufügen oder Abtragen von Material oder Materialkomponenten.

Die Probenanordnungsbereich 31 weist Stege 32, 33 auf, die einen Spalt 34 begrenzen, in dem Probenmaterial 35 angesammelt wird.

Zur Unterstützung der Ansammlung von Probenmaterial 35 in dem Spalt 34 sind Elektroden 36, 37 vorgesehen, über die Zellen oder sonstiges Probenmaterial in dem Spalt 34 aufkonzentriert werden, die über mikrofluidische Zufuhrkanäle 38 in den Probenanordnungsbereich 31 geleitet werden.

Den Zufuhrkanälen 38 gegenüberliegend sind mikrofluidische Ausleitkanäle 39 vorgesehen, über die durchgespültes Medium sowie ggf. entnommene flüssige Messproben ausgeleitet werden.

Ein Probenanordnungsbereich 31 kann mit je einem Zufuhrkanal 38 und Ausleitkanal 39 oder mit mehreren Zufuhrkanälen 38 und Ausleitkanäle 39 verbunden sein.

Die Zufuhrkanäle 38 sind mit einem mikrofluidischen Funktionsbereich 41 und die Ausleitkanäle 39 mit einem mikrofluidischen Funktionsbereich 42 verbunden.

Diese Funktionsbereiche 41, 42 umfassen beispielsweise Ventilanordnungen und Verzweigungsstrukturen, um für eine gleichmäßige Verteilung und/oder einen selektiv gesteuerten Fluss der flüssigen Medien zu sorgen. Hier sind für die Steuerung flüssiger Medien bspw. Kapillarstopventile und für die Steuerung gasförmiger Medien Sperrventile vorgesehen.

Über mikrofluidische Kanäle 43 und 44 sind die Funktionsbereiche 41 und 42 mit dem Anschlussbereich 16 versehen. Es versteht sich, dass die Kanäle 43 und 44 mehrere parallele, voneinander fluidisch getrennt verlaufende mikrofluidische Kanäle umfassen können, so dass über den Anschlussbereich 16 unterschiedliche Medien, insbesondere Gase, Substanzen und Flüssigkeiten in den Probenanordnungsbereich 31 geleitet und aus diesem ausgeleitet werden können.

Der Anschlussbereich 16 enthält nicht gezeigte Anschlusselemente, die dem Zufuhr/der Abfuhr flüssiger Medien, bspw. zum Transport der biologischen Probe oder zu testender Substanzen, und gasförmiger Stoffe, bspw. Prozessgase und Aerosole, dienen. Zu diesen Anschlusselementen können auch Pippetieröffnungen für gravitationsgetriebenen Fluss zählen, die von Pippetiersystemen bedient werden.

In dem Anschlussbereich 16 sind ferner Anschlusselemente für die Weiterleitung elektrischer Signale und für über Lichtwellenleiter transportierte optische Signale oder Membranen zur Übertragung von Druck oder akustischen Schwingungen vorgesehen.

Zwischen dem Anschlussbereich 16 und dem mikrofluidischen Funktionsbereich 41 ist in dem Kanal 43 noch eine Funktionseinheit 45 vorgesehen, die beispielsweise eine Blasenfalle sein kann und dafür sorgt, dass keine Gasblasen in den Probenanordnungsbereich 31 hineingelangen, weil diese dort die Verteilung von flüssigem Medium behindern würden.

Die Blasenfalle 45 trennt gasförmige Anteile aus dem zugeführten Medienstrom. Sie kann als aktives Element mit vakuumunterstützter Absaugung mit oder ohne Sperrmittel sowie als rein passives Element mit speziellen Membranen ausgestaltet sein.

Die Elektroden 36 und 37 sind über elektrische Leitungen 46 und 47 mit elektrischen Funktionsbereichen 48 und 49 verbunden, die jeweils über elektrische Ein- oder Mehrfachleitungen 51 und 52 mit dem Anschlussbereich 16 verbunden sind.

Die elektrischen Funktionsbereichen 48 und 49 dienen der aktiv gesteuerten oder passiven Verteilung und Sammlung von elektrischen Signalen.

In der Probenkanaleinheit 15 bzw. dem Probenanordnungsbereich 31 sind ferner verschiedene optisch empfindliche, elektrische, elektrochemische oder biosensorische Funktionselemente 53, 54 und 55 vorgesehen, über die optisch empfindliche, elektrische oder chemische Signale zur Überprüfung, Steuerung oder Messwerterfassung abgefragt und an dem Anschlussbereich 16 bereitgestellt werden.

Diese lokale Sensorik im Probenanordnungsbereich 31 und in der Probenkanaleinheit 15 erlaubt es, Messwerte für Aussagen über die Vitalität von in die Probenkanaleinheit 15 eingespülten und/oder im Probenanordnungsbereich 31 assemblierten Zellen zu erhalten. Dazu werden z.B. integrierte Sensoren 53, 54, 55 verwendet, mit denen zum Beispiel die O₂/CO₂-Ionenkonzentration, der pH-Wert, Impedanzen oder Metabolismusprodukte gemessen werden können.

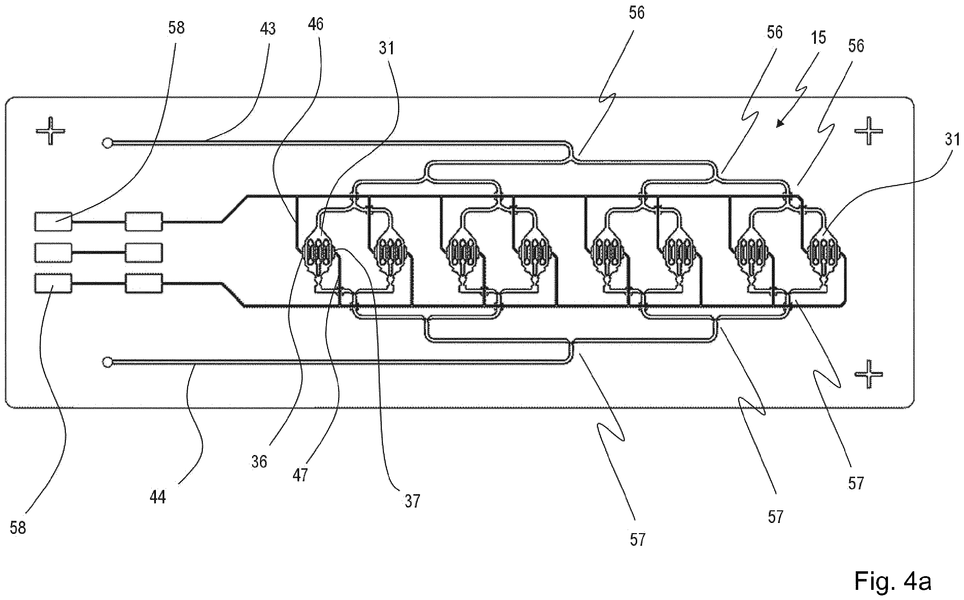

Fig. 4a zeigt den prinzipiellen Aufbau einer Probenkanaleinheit 15, in der insgesamt acht Probenanordnungsbereiche 31 fluidisch und elektrisch parallel geschaltet sind. Zu diesem Zweck wird ein mikrofluidischer Zufuhrkanal 43 mit Hilfe von Aufteilungsstellen 56 mehrfach aufgeteilt.

In entsprechender Weise ist der mikrofluidische Ausleitkanal 44 über Kombinationsstellen 57 mit den Probenanordnungsbereichen 31 fluidisch verbunden.

In Fig. 4a sind ferner die Elektroden 36 und 37 der einzelnen Probenanordnungsbereiche 31 zu erkennen, die elektrisch parallel geschaltet sind und über gemeinsame Anschlusselemente 58 angesteuert werden.

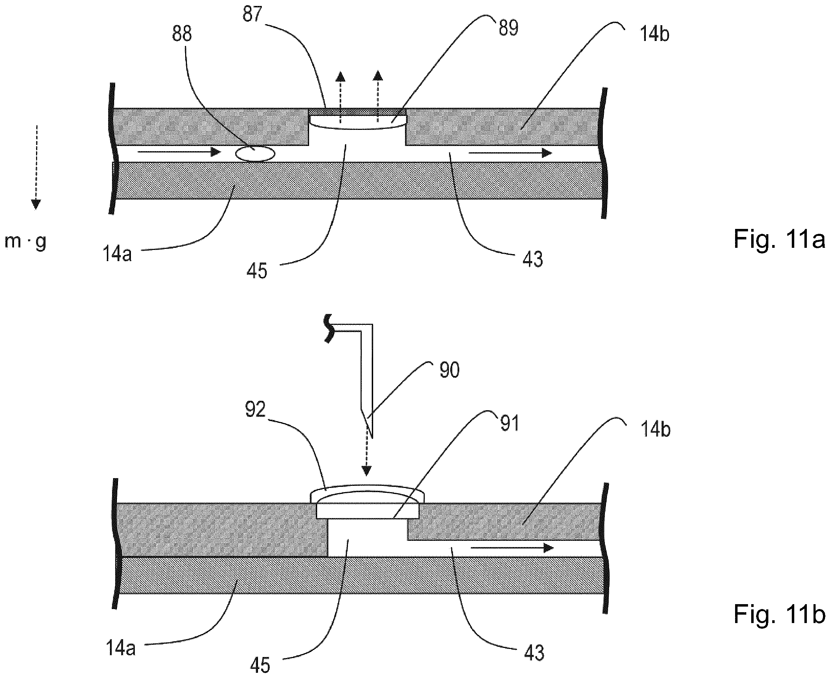

Nicht gezeigt in Fig. 4a ist die obere Abdeckung, die auf dem Trägersubstrat 14a des Probenchips 14 aufliegt und die nach oben offenen Mikrostrukturen abdeckt. Wie ein derartiges gedeckeltes mikrofluidisches System hergestellt werden kann, ist in der eingangs erwähnten DE 10 2008 018 170 A1 beschrieben, auf deren Inhalt hiermit Bezug genommen wird. Nachstehend zeigt Fig. 11a eine Querschnittsdarstellung durch einen Probenchip 14, in der das Trägersubstrat 14a und ein Kanaldeckel 14b zu erkennen sind.

Dieser Deckel 14b ist zumindest bereichsweise optisch dünn ausgelegt, so dass das Probenmaterial in dem Probenanordnungsbereich mit optischen Verfahren analysiert werden kann.

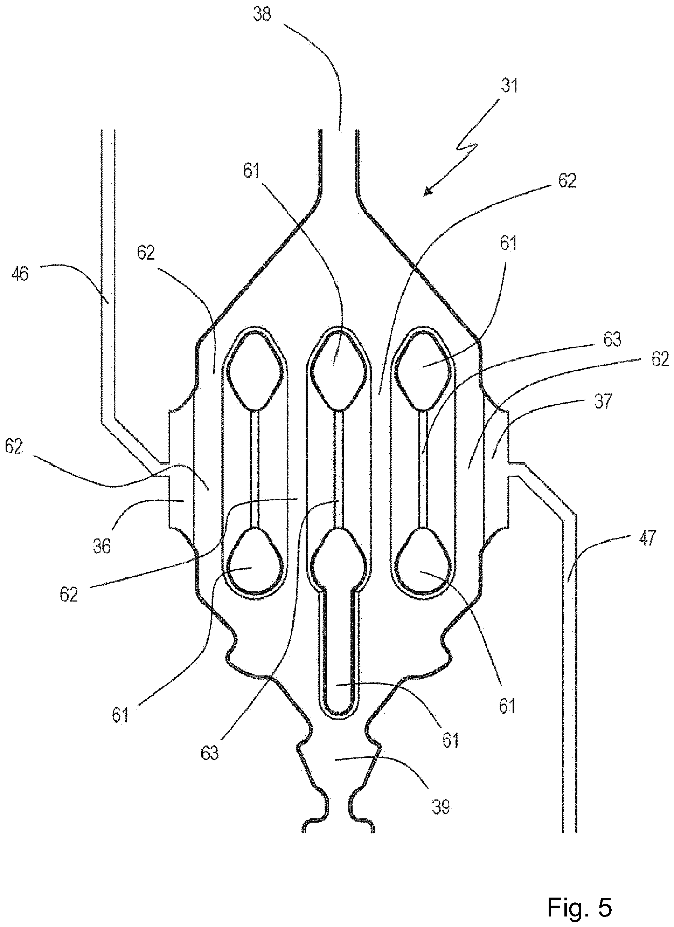

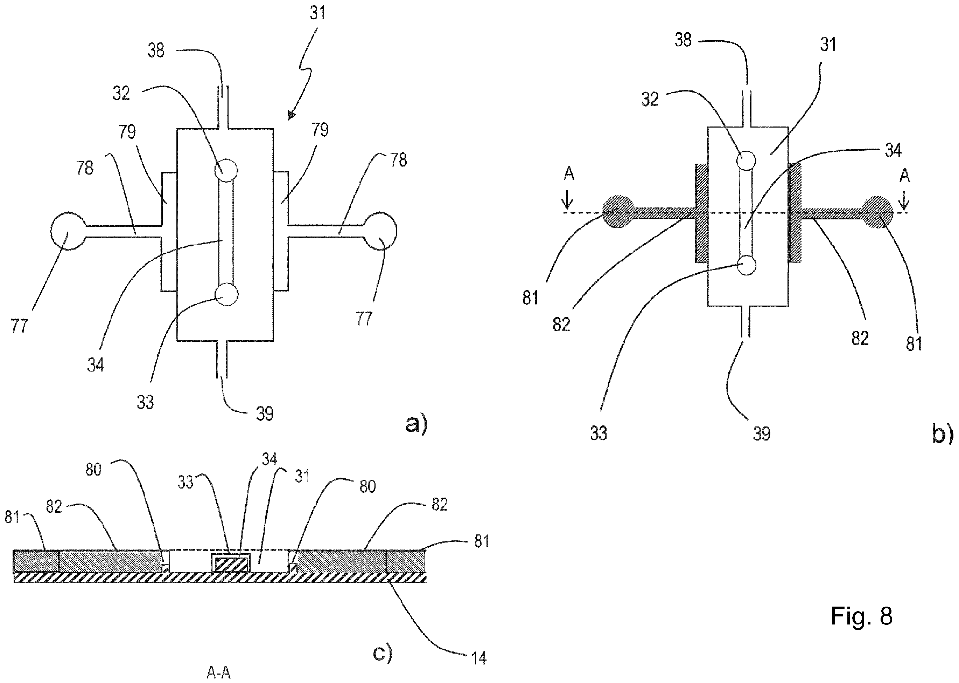

Fig. 5 zeigt eine vergrößerte Draufsicht auf einen Probenanordnungsbereich 31, der aus mehreren Stegen 61 und mikrofluidischen Kanälen 62 besteht, die zwischen den Elektroden 36, 37 angeordnet und über Öffnungen 63 miteinander verbunden sind. Die Stege 61 und die Öffnungen 63 sind Beispiele für die Stege 32, 33 und den Spalt 34 aus Fig. 3; siehe auch Fig. 8c.

Der Probenanordnungsbereich 31 ist ein speziell geformter Bereich der Probenkanaleinheit 15, in dem das biologische Probenmaterial 35, insbesondere biologische Zellen und Zellbestandteile, unter Einwirkung äußerer Kräfte, insbesondere elektrische Feldstärke oder Flussbedingungen, gezielt angeordnet werden kann.

Zwischen den Stegen 61 sind die Öffnungen 63 vorgesehen, in/an denen biologisches Probenmaterial unter der Wirkung elektrischer Felder, die durch die Elektroden 36, 37 erzeugt werden assembliert werden kann, wie es in den eingangs erwähnten DE 10 2008 018 170 B4 sowie DE 10 2009 039 956 A1 beschrieben ist.

Durch die Strukturierung der Kanäle 62 und Stege 61 kann das zwischen den Elektroden 36, 37 anliegende elektrische Feld in seiner Wirkung so manipuliert werden, dass die Feldstärke im Bereich der Öffnungen 63 gezielt erhöht oder abgeschwächt wird, um die Assemblierung des biologischen Probenmaterials 35 zu beeinflussen.

Die Oberflächen der Stege 61 und Kanäle 62 können dabei gezielt modifiziert sein, um für die jeweilige biologische Probe eine den natürlichen Umgebungsbedingungen entsprechende Umgebung zu schaffen. Diese Modifikationen können eine Hydrophilisierung oder Hydrophobisierung der betreffenden Oberflächen umfassen.

Durch die Geometrie der Stege 61 und Kanäle 62 wird ferner die Strömungsgeschwindigkeit beeinflusst, um die Assemblierung der biologischen Probe zu unterstützen. Durch Querschnittserweiterungen in den Kanälen 62 können beispielsweise strömungsberuhigte Bereiche geschaffen werden.

Die Elektroden 36, 37 sind dabei besonders strukturierte, leitfähige Flächen, die unter Anlegung elektrischer Gleich- und/oder Wechselspannungen einerseits elektrische Felder zur Beeinflussung der Assemblierung des biologischen Probenmaterials entstehen lassen.

Andererseits erlaubt der bei Anlegung elektrischer Gleich- und/oder Wechselspannungen gemessene Stromfluss Rückschlüsse auf die Impedanz von Substanzen im Probenanordnungsbereich 31, so dass die Elektroden 36, 37 auch als Sensoren wirken. Ferner können über die Elektroden 36, 37 elektrische Pulse erzeugt werden, mit denen die in dem Probenanordnungsbereich assemblierten Zellen aufgeschlossen werden können.

Durch die Parallelschaltung von hier acht Probenanordnungsbereichen 31 kann in einer Probenkanaleinheit 15 identisches Probenmaterial mit verschiedenen Substanzen getestet werden. Alternative kann in den unterschiedlichen Probenkanaleinheiten 15 unterschiedliches biologisches Probenmaterial assembliert und mit derselben Substanz getestet werden, die durch den Zufuhrkanal 38 zugeführt wird.

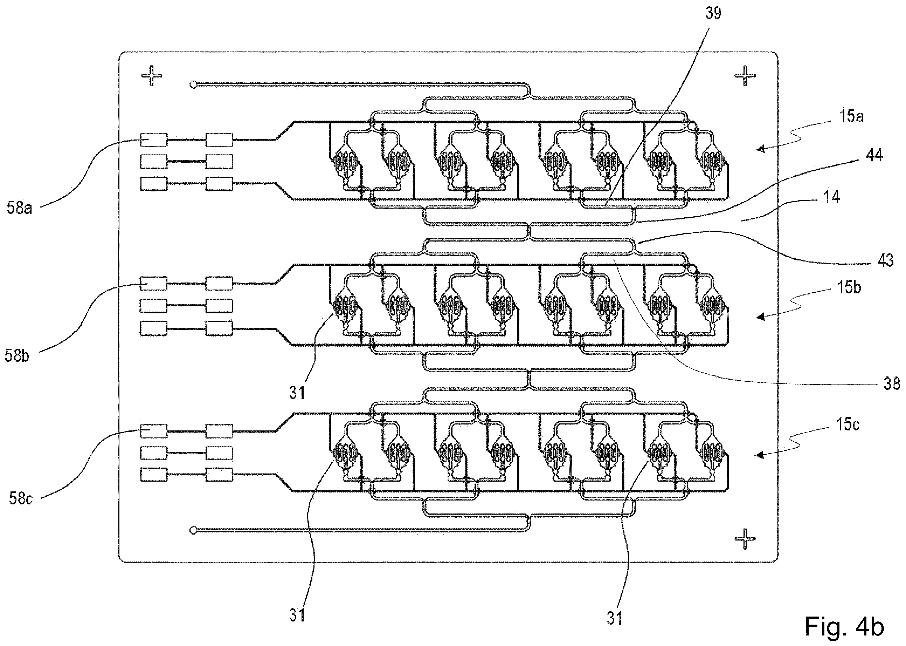

Es ist auch möglich, auf einem Probenchip 14 zwei oder mehr Probenanordnungsbereiche 31 oder Probenkanaleinheiten 15 in Reihe zu schalten, so dass der Ausleitkanal 39 eines in Flussrichtung ersten Probenanordnungsbereiches 31 in den Zufuhrkanal 38 eines fluidisch nachgeschalteten Probenanordnungsbereiches 31 führt. Die Elektroden 36, 37 der beiden Probenanordnungsbereiche 31 sind dann unabhängig voneinander ansteuerbar und auslesbar. Fig. 4b zeigt einen Probenchip 14, auf dem 3 Probenkanaleinheiten 15a, 15b und 15c in Reihe zueinander geschaltet sind.

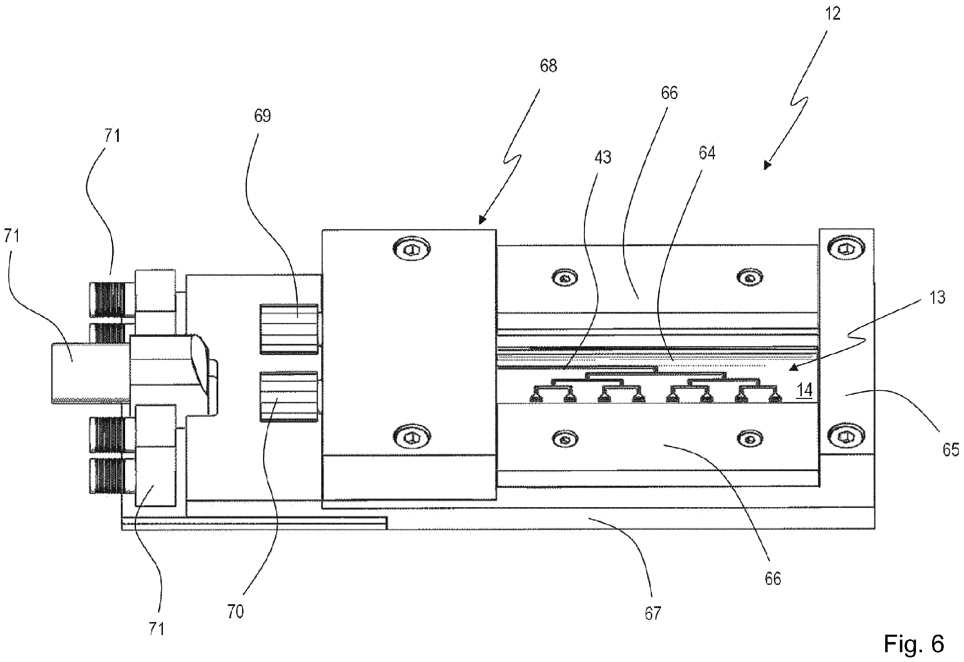

Fig. 6 zeigt eine perspektivische Draufsicht auf ein Ausführungsbeispiel für die Anschlussplatte 12 aus Fig. 1.

Rechts in Fig. 6 ist in dem Aufnahmebereich 13 ein Probenchip 14 zu erkennen, auf dem eine optisch dünne Abdeckplatte oder Abdeckfolie 64 aufliegt, die zusammen mit dem Probenchip 14 über Spannelemente 65, 66 auf einen Träger 67 der Anschlussplatte 12 aufgespannt ist.

Links neben dem Probenchip 14 befindet sich ein dem fluidischen Anschluss dienender Fluidikblock 68, der im vorliegenden Fall ein mit dem Zulaufkanal 43 kommunizierendes Zulaufelement 69 sowie ein mit dem in Fig. 6 nicht zu sehenden Ausleitkanal 44 kommunizierendes Ablaufelement 70 umfasst.

Links außen an der Anschlussplatte 12 sind elektrische Anschlusselemente 71 zu erkennen, die mit den Leitungen 46, 47 kommunizieren.

Die Elemente 69, 70, 71 sind äußere Anschlusselemente 23 der Anschlussplatte 12.

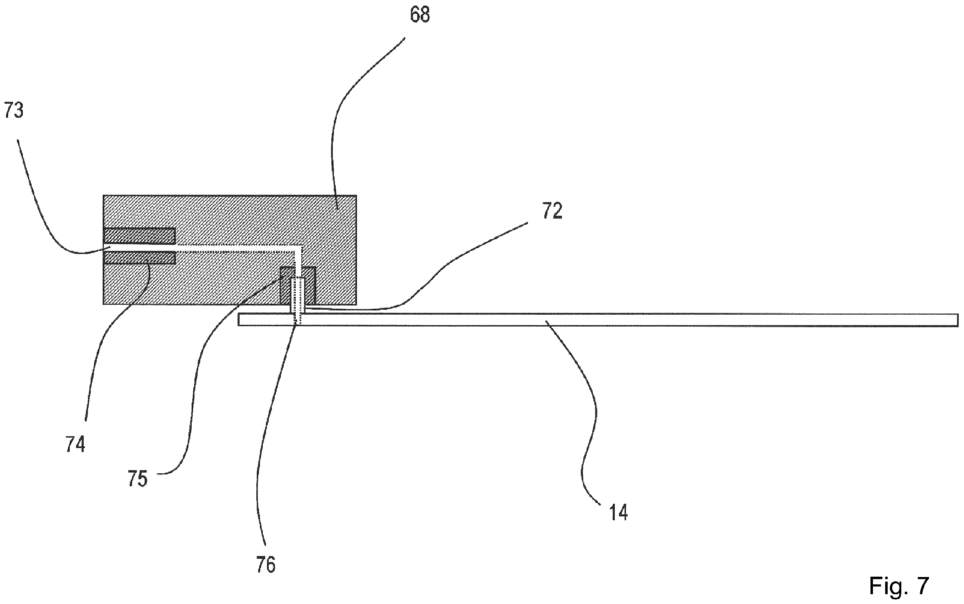

Fig. 7 zeigt einen Längsschnitt durch den Fluidikblock 68 der Anschlussplatte 12 aus Fig. 6, aus dem sich die fluidische Kontaktierung des Probenchips 14 ergibt. Der Fluidikblock 68 dient zur Aufnahme von Dichtungselementen und Anschlusskanälen. Er ist beispielsweise aus Polycarbonat oder Polymethylmethacrylat gefertigt.

Dabei bezeichnet 72 einen als Anschlussolive ausgebildeten, vertikalen Anschluss auf dem Probenchip 14, und 73 eine der Verbindung zum Außenanschluss dienende Kanalstruktur, die endseitig von einem Dichtungselement 74 umgeben ist, das zum Anschluss von Zulauf- und Ablaufelementen 69, 70 dient und als Schraub- oder Klemmverbindung ausgebildet sein kann.

Ein vergleichbares Dichtungselement 75 ist dem vertikalen Anschluss 72 zugeordnet, der die Verbindung zu den probenchipseitigen Kanalstrukturen 76 herstellt, die beispielsweise durch die Zufuhrkanäle 38 und Ausleitkanälen 39 gebildet wird.

Das Element 75 entspricht dabei einem der in Fig. 1 gezeigten inneren Anschlusselemente 21 der Anschlussplatte 12. Die Elemente 72, 76 sind ein Beispiel für die in Fig. 2 gezeigten Anschlusselementen 18 des Probenchips 14.

Während die Elektroden 36 und 37 durch Goldelektroden ausgebildet sein können, zeigt Fig. 8 ein Ausführungsbeispiel, bei dem die Elektroden durch leitfähiges Ter-Polymer gebildet sind, wie dies beispielsweise in der nicht vorveröffentlichten DE 10 2012 102 321 beschrieben ist.

Dadurch werden die mit der Herstellung von Goldelektroden verbundene Probleme hinsichtlich der genauen Positionierung der Maske für die Elektroden vermieden. Die Polymerelektroden werden durch Einleiten von auspolymerisierenden Monomeren in entsprechende Mikrokanäle gebildet, wie es beispielsweise ausführlich in der nicht vorveröffentlichten DE 10 2012 102 321 beschrieben ist.

Demgemäß sind die Elektroden als Festkörperelektrolyt ausgebildet, der eine Polymermatrix umfasst, die hergestellt wird durch Polymerisieren einer Monomerlösung, die zumindest ein Monomer, ggf. zumindest einen Quervernetzer, und zumindest eine direkt polymerisierbare ionische Flüssigkeit aufweist, die einen organischen Ladungsträger, vorzugsweise organisches Kation, und einen anorganischen Gegenladungsträger, vorzugsweise ein anorganisches Anion, aufweist, wobei das organische Kation vorzugsweise zumindest einen Allyl-Rest, weiter vorzugsweise zumindest einen quarternären Stickstoff aufweist.

Unter einer direkt polymerisierbaren ionischen Flüssigkeit wird eine ionische Flüssigkeit verstanden, die ohne vorherige Derivatisierung des organischen Ladungsträgers eingesetzt werden kann.

Zur Herstellung der Elektroden wird eine Monomerenlösung verwendet, in der zunächst drei Monomere in einer organischen Lösung, bspw. Isopropanol, vorliegen. Die Monomerlösung besteht aus einem einfachen Acrylat (einfache Ketten), welches bei alleiniger Polymerisation lineare Ketten ergibt und die Grundstruktur des Polymers darstellt (hier: HEMA - 2-Hydroxyethyl 2-methylprop-2-enoat). Dazu kommt ein Bisacrylat (hier: EGDMA - 2-(2-Methyl-acryloyloxy)ethyl-2-methyl-acrylat), welches die Quervernetzung gewährleistet (doppelte, verlinkte Ketten). Als letztes Monomer wird eine ungesättigte Ionische Flüssigkeit (hier: AlMeImCl - 1-Allyl-3-methylimidazolium-chlorid) dazugegeben. Dieses besteht aus Chloridionen und einem großen, positiv geladenen organischen Kation. In einer Ausführung werden die Monomere durch eine kontrollierte freie radikalische Polymerisation mittels UV Initiator (hier: Irgacure2959 - 2-Hydroxy-4'-(2-hydroxyethoxy)-2-methylpropiophenon) zu einem Ter-Polymer polymerisiert.

Alternativ ist es auch möglich, als Elektrodenpolymer eine elektrisch leitfähige Monomerenlösung in die Mikrokanäle 77, 78, 79 einzufüllen und aushärten zu lassen.

Fig. 8a zeigt eine schematische Draufsicht auf einen Probenanordnungsbereich 31 mit Stegen 32, 33 und Spalt 34 sowie mikrofluidischem Zufuhrkanal 38 und Ausleitkanal 39. Mit 77 sind Einleitöffnungen für das Elektrodenpolymer bezeichnet, die mit Leitkanälen 78 kommunizieren, die zu Strukturierungsbereichen 79 führen, in denen die Polymerelektroden ausgebildet werden.

Fig. 8b zeigt eine Darstellung wie Fig. 8a, jedoch mit mit Elektrodenpolymer gefüllten Mikrokanälen 77, 78, 79. Fig. 8c zeigt eine Schnittdarstellung längs der Linie A-A aus Fig. 8b, in der vertikale Kapillarstopps 80 für das Elektrodenpolymer zu erkennen sind. Die Kapillarstopps 80 dienen dazu, das eingefüllte Elektrodenpolymer entlang des Strukturierungsbereiches 79 zu führen, so dass Elektrodenpolymer nicht in den Probenanordnungsbereich 31 gelangen kann.

81 bezeichnet für die externe Kontaktierung in der Einleitöffnung 77 ausgebildete Kontaktflächen und 82 die in dem Strukturierungsbereich 79 ausgebildete Polymerelektrode.

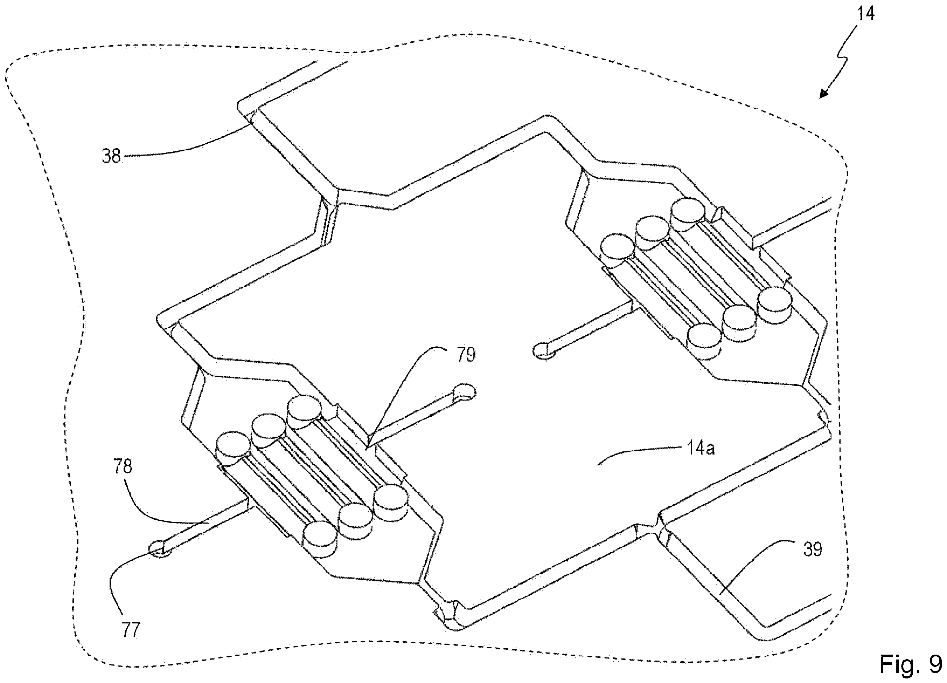

Die erforderlichen Mikrokanäle werden zusammen mit der übrigen Mikrostrukturierung des Probenchips im Spritzgussverfahren hergestellt, so dass auf Metallisierung verzichtet werden kann. In Fig. 9 ist ausschnittsweise ein Beispiel für einen Probenchip 14 gezeigt, in dem die Mikrofluidikkanäle 38, 39 zusammen mit den Mikrokanälen 77, 78, 79 und der übrigen Mikrostrukturierung in einem Spritzgussfertigungsschritt hergestellt wurden.

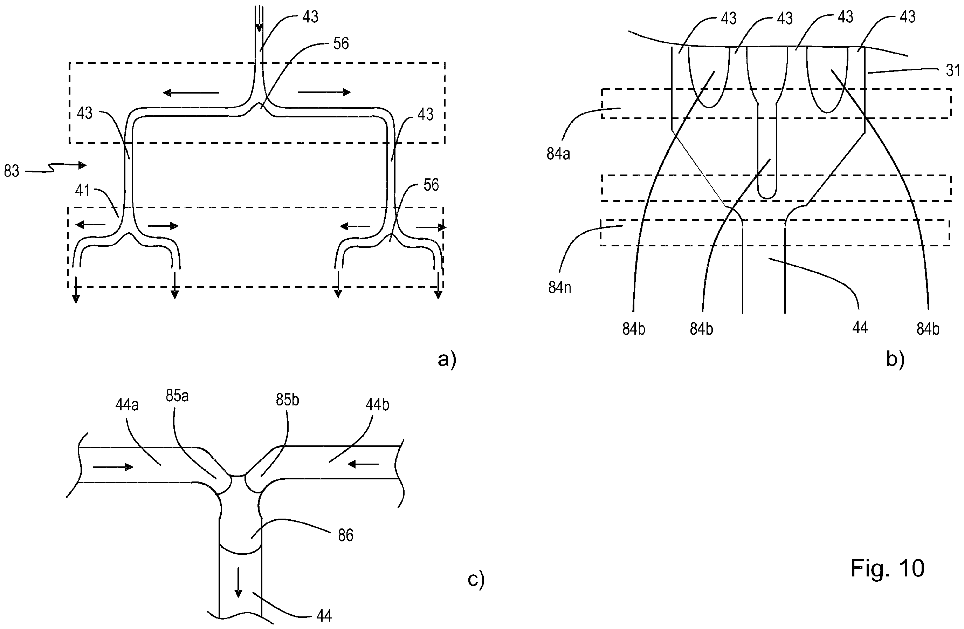

Fig. 10 zeigt Beispiele für verschiedene Ventilsysteme und Aufteilungsstellen, über die dafür gesorgt wird, dass sich sämtliche mikrofluidischen Kanäle 43, 44 in der in Fig. 4 gezeigten Probenkanaleinheit 15 mit Flüssigkeit füllen.

In mikrofluidischen Probenchips 14 aus hydrophoben Materialien mit mehreren sich verzweigenden Mikrokanälen 43, 44 stellt die gleichmäßige Befüllung aller Mikrokanäle 43, 44 aufgrund der hohen Oberflächenspannung oft ein Problem dar; siehe Fig. 10a. Besonders wenn die Mikrokanäle 44 wieder zusammengeführt werden, können Luftblasen eingeschlossen werden, die sich auch kaum mehr entfernen lassen.

Durch Aneinanderreihung einer Kaskade von Multiplexerstrukturen und Kapillarstopventilen mit zunehmenden Berstdrücken ist es dennoch möglich, einen solchen Probenchip 14 luftblasenfrei zu befüllen.

Eine Kaskade 83 besteht aus einem oder mehreren Multiplexern (Fig. 10a), an denen die Mikrokanäle 43 mittels der Aufteilungsstellen 56 separiert werden. Eine Aufteilungsstelle 56 teilt einen eingehenden Flüssigkeitsstrom an seiner Phasengrenze in zwei neue Phasengrenzen, ohne jedoch abrupten Druckänderungen zu unterliegen. Figur 10a zeigt eine mögliche Ausführungsform eines solchen Funktionsbereiches.

Ferner sind kaskadierte Kapillarstoppventile (Fig. 10b) für eine gleichmäßige Befüllung der Mikrokanäle 43, 44 und der Probenanordnungsbereiche 31 vorgesehen. Hierbei stoppt ein Flüssigkeitsstrom mit seiner Phasengrenze zunächst an einem Kapillarstoppventil einer ersten Kaskadenstufe 84a, die Strömungshindernisse 84 b enthält, an denen die Mikrokanäle 43 zusammengeführt werden. Erst wenn Flüssigkeitsströme aus sämtlichen Mikrokanälen 43 eines Probenaufnahmebereiches an den Kapillarstoppventilen der ersten Kaskadenstufe anliegen, wird der Berstdruck überschritten und die Menisken der einzelnen Phasengrenzen werden zusammengeführt. Dieser Vorgang wiederholt sich für die nachfolgenden Kaskadenstufen solange, bis die Flüssigkeitsströme sämtlicher Probenbereiche an der letzten Kaskadenstufe 84n anliegen.

Weiter sind ein oder mehrere Merger-Ventile (Fig. 10c) oder Kaskaden von Merger-Ventilen vorgesehen, an denen die Mikrokanäle 44a, 44b wieder zusammengeführt werden. Die Merger-Ventile "schalten" erst durch, wenn Flüssigkeitsströme aus beiden Mikrokanälen 44a, 44b anliegen und die Flüssigkeitsmenisken 85a, 85b sich berühren und dadurch einen einzelnen Folgemeniskus 86 mit größerem Radius und mithin kleinerem Berstdruck bilden (p ist proportional zu γ/2R, mit γ = Oberflächenspannung des Mediums, R = Krümmungsradius des Meniskus).

Verwendbare Ventile sind beispielsweise beschrieben in EP 1 441 131 A1 und US 6,601, 613 B2.

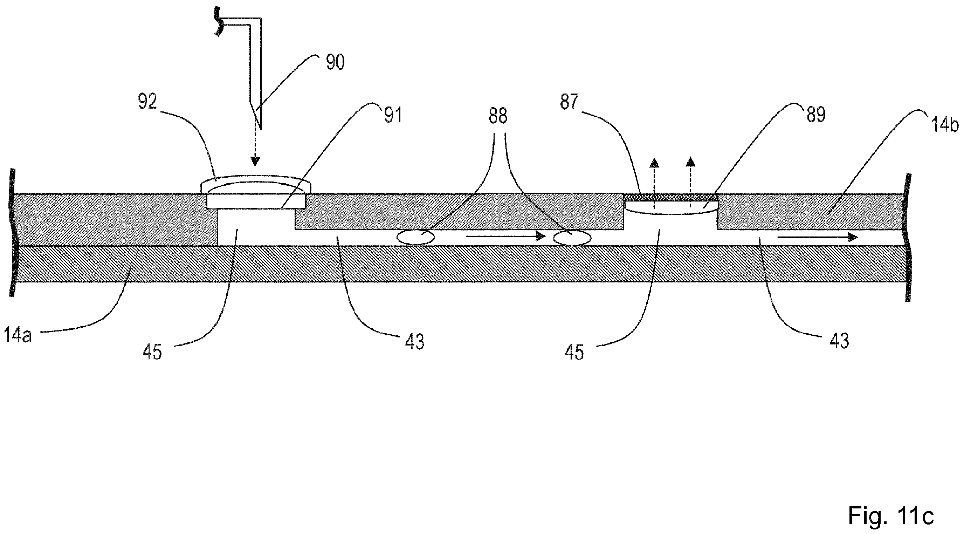

Fig. 11 zeigt Beispiele, um für ein blasenfreies Füllen der mikrofluidischen Kanäle 43 zu sorgen. In Fig. 11a bezeichnet 45 eine als Blasenfalle ausgebildete Funktionseinheit.

In Fig. 11a bezeichnet ferner 14a das Trägersubstrat und 14b den Kanaldeckel für einen Mikrokanal 43.

Gelangen Luftblasen 88 in einen mikrofluidischen Probenchip 14, ist dieser meist nicht mehr funktionstüchtig. Zudem können Luftblasen 88 die Zellen im Probenchip 14 schädigen. Daher kann der eigentlichen Fluidik mindestens eine Blasenfalle 45 vorgeschaltet sein. Diese lässt sich direkt in den Probenchip 14 oder in die Anschlussplatte 12 integrieren, wie dies beispielhaft in Fig. 11a und 11c gezeigt ist.

Dazu ist beispielsweise gemäß der Querschnittsdarstellung in Fig. 11a eine gaspermeable Membran 87 vorgesehen, die in den Kanaldeckel 14b eingelassen ist. In einer Aushöhlung am Kanaldeckel 14b werden Luftblasen 88 aus dem Kanal 43 abgefangen und das Gas 89 entweicht durch die Membran 87. Bei dieser Ausführung handelt es sich um eine rein passive Blasenfalle. Wird zusätzlich eine unterstützende Vakuumabsaugung eingesetzt, handelt es sich um eine aktive Blasenfalle.

Weiterhin sind parallelisierte Verschaltungen sowie Kaskaden derartiger passiver Blasenfallen denkbar.

Wie bereits erwähnt, wird als Kontrolleinheit 11 entweder eine Dielektrophoreseeinheit oder eine Perfusionseinheit eingesetzt. Während die Ansammlung der Zellkultur nämlich nur wenige Minuten dauert, soll die Kultivierung der Zellen über mehrere Wochen erfolgen. Wird beides mit derselben Kontrolleinheit 11 durchgeführt, ist diese für mehrere Wochen blockiert, bevor erneut eine Assemblierung von Zellen stattfinden kann.

Die Dielektrophoreseeinheit steuert das Priming des Chips, die Ansammlung der Zellen mittels Dielektrophorese und den Aufbau der Zellkultur. Die Perfusionseinheit sorgt dann für die Versorgung der Zellkultur mit Medium und regelt die Inkubation mit Testsubstanzen über die Kulturzeit von Tagen bis Wochen.

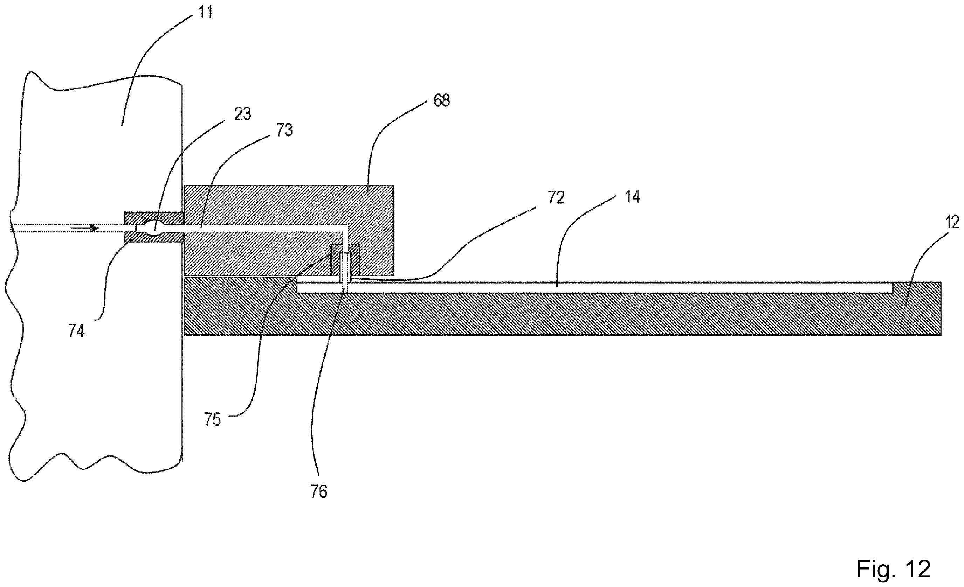

Die Kontaktierung des Probenchips 14 zu den beiden Kontrolleinheiten 11 erfolgt über die Anschlussplatte 12 und muss die blasen- und kontaminationsfreien Umkopplung, also das Ab- und spätere Wiederankoppeln des Probenchips von einer Kontrolleinheit zur anderen erlauben. Diese Manipulationen führen praktisch unvermeidbar zum Eindringen von Luftblasen 88 von den äußeren Anschlusselementen 23 der Anschlussplatte 12 in den Probenchip 14; siehe Fig. 12. Diese Luftblasen stören jedoch den Fluss, bspw. durch Veränderung des Fließwiderstandes im betroffenen Kanalsegment, und zerstören die Zellkultur, wenn sie in die Probenanordnungsbereiche gelangen.

Die Kontaktierung kann gemäß Fig. 11b über ein Septum 91 auf dem Probenchip 14 oder der Anschlussplatte 12 erfolgen, wobei die Verbindung zu der Kontrolleinheit 11 durch eine Hohlnadel 90 hergestellt wird. Die Hohlnadel 90 kann Bestandteil der Kontrolleinheit 11 aber auch Bestandteil der Anschlussplatte 12 sein.

Die luftblasenfreie Umkopplung erfolgt vorzugsweise jedoch über eine mikrofluidische Entlüftungsvorrichtung gemäß Fig. 11a erweitert um ein Septum 91 gemäß Fig. 11b, wie es in Kombination in Fig. 11c dargestellt ist. Über die Hohlnadel 90 beim An- oder Umkoppeln in die Mikrokanäle 43 eindringende Luftblasen 88 werden in der stromabwärts liegenden Blasenfalle über die Membran 87 wieder ausgeschleust.

Das Septum 91 kann vor Kontamination geschützt sein, vorzugsweise durch eine Blisterfolie 92, welche das Septum 91 zumindest teilweise abdeckt.

Die Ankopplung der Anschlussplatte 12 an die Kontrolleinheiten 11 kann über Zulaufelemente 69 und Ablaufelemente 70 als Kombinationen von Schlauch und Schraubverbindungen erfolgen. Eine Entgasung erfolgt wie bereits oben erwähnt.

Bevorzugt wird jedoch die Ankopplung der Anschlussplatte über ein Stecksystem wie es beispielhaft in Fig. 12 dargestellt ist, bei dem das Umkoppeln des Probenchips völlig ohne Schlauchmaterial ausgeführt werden kann.

In Fig. 12 ist in einer Schnittdarstellung die Ankopplung eines auf eine Anschlussplatte 12 aufgeklemmten Probenchips 14 an eine Kontrolleinheit 11 über den aus Fig. 7 bekannten Fluidikblock 68 und äußere Anschlusselemente 23 gezeigt. Das Dichtungselement 74 sitzt hier in der Kontrolleinheit 11, ein Anschlusselement 23 steckt abgedichtet in diesem Dichtungselement 74. Der Anschluss des Fluidikblocks 68 an den Probenchip 14 erfolgt wie in Fig. 7, die Entlüftung wie in Fig. 11a.

Die Integration der Funktionselemente 41, 42, 45, 48, 49, 53, 54, 55 in die einzelnen Probenkanaleinheiten 15 ermöglicht eine Überwachung verschiedener Parameter, wie beispielsweise Elektrodenfunktion, Befüllung und Luftblasenfreiheit des Probenchips 14, Flussraten, Temperatur, Gasgehalt der Medien, Dichte der Zellkultur und Zellzahl etc., ohne dass optische Überwachungen und händische Schritte erforderlich sind.

Geeignete Messprinzipien dafür sind beispielsweise die Impedanzmessung für Flussraten, Luftblasen, Zelldichte und Steuerung der Zellassemblierung, sowie optische/elektrochemische O₂/CO₂-Sensoren, Temperatursensoren und Dehnungsmessstreifen zur Erfassung von Flussraten und Luftblasen.

Ferner können optoelektronische Sensoren oder mikrooptische bzw. optofluidische Verfahren zum Einsatz kommen, wie es z.B. beschrieben ist in der US 2011/0181884 A1.

Impedanzmessungen können dabei auch über die Elektroden 36 und 37 vorgenommen werden.

Die insoweit beschriebenen Funktionselemente 41, 42, 45, 48, 49, 53, 54, 55 dienen der Qualitätssicherung, also der Funktionalität des Probenchips 14, der Überwachung der Zelldichte und vor allem auch der Kalibrierung des gesamten Systems.

Darüber hinaus können die Funktionselemente auch zur Messung der Zellreaktion, also beispielsweise zur Messung der Vitalität oder der Aktivität bei Zufuhr von chemischen Substanzen oder Medikamenten verwendet werden.

Während im Stand der Technik die Messung der Zellantwort bisher optisch über Fluoreszenzmikroskopie und chemisch durch Analyse des Perfundats erfolgte, erlauben die integrierten Sensoren, also die Funktionselemente 53, 54, 55, eine weniger aufwendige und damit preiswertere und vor allem kontinuierliche Messung der Zellreaktionen.

Dabei kann beispielsweise über die Messung des Sauerstoffgehalts im Medium bevor und nachdem das Medium die Probenanordnungsbereiche 31 passiert hat, die Bestimmung der Zellvitalität erfolgen.

Messungen der Impedanz der Zellanordnung im Probenanordnungsbereich 31 geben dabei beispielsweise eine Aussage über die Dichtigkeit von Gewebebarrieren, wie sie beispielsweise in der Blut-Hirn-Schranke, im Darm oder in der Niere zu finden sind.

Dabei geben die auf dem Probenchip 14 direkt, also sozusagen on-line durchgeführten kontinuierlichen Messungen von lonenkonzentration, pH-Wert oder von Produkten des Zellmetabolismus im Medium direkt Aufschluss über die Funktionalität der Zellen, ohne dass das Medium hinter dem Probenchip 14 aufgefangen und analysiert werden muss. Diese Messungen können elektrochemisch oder mit Hilfe von Biosensoren durchgeführt werden, die immobilisierte Antikörper und/oder Enzyme verwenden.

Um komplexere, asymmetrische Gewebestrukturen wie beispielsweise Endothelbarrieren des Darms oder der Blut-Hirn-Schranke aufbauen zu können, ist es erfindungsgemäß vorgesehen, im Bereich des Spaltes 34, also in dem Bereich zwischen den Mikrokanälen 62, wo sich das biologische Probenmaterial 35 anordnet, eine Trennstruktur 94 anzuordnen, die als Hydrogel 97 ausgebildet sein kann.

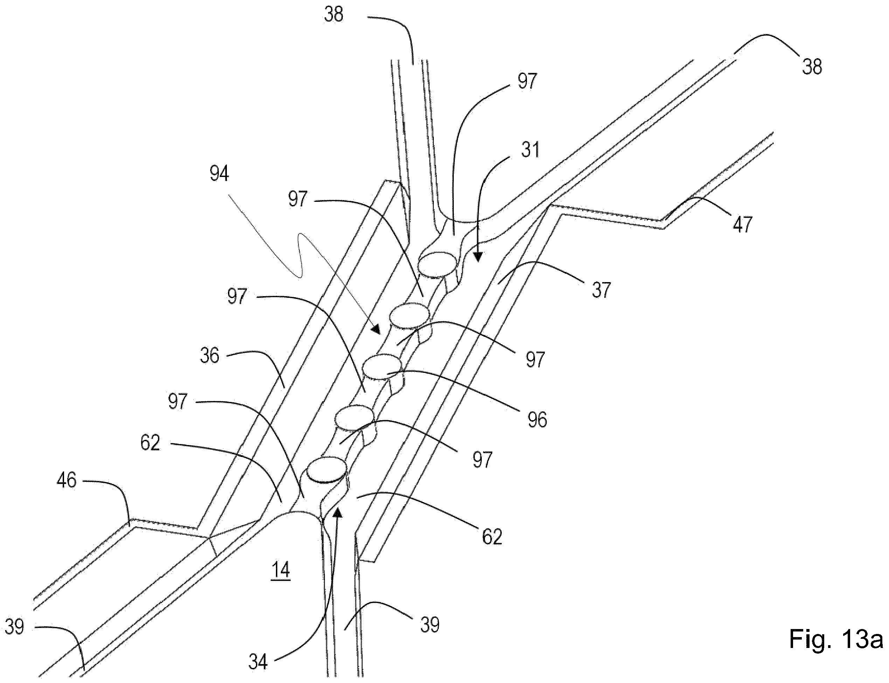

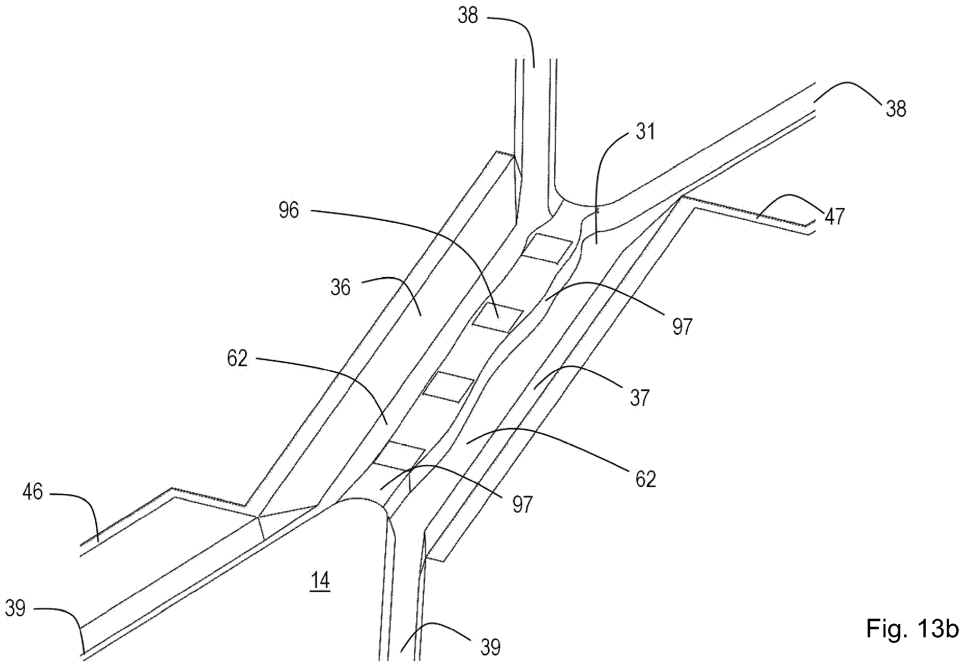

Das Hydrogel kann zwischen Pfosten 96 angeordnet sein, die im Spalt 34 vorgesehen sind, wie es in Fig. 13 gezeigt ist, wo das Hydrogel den Spalt 34 und die Pfosten 96 gemäß Fig. 13a teilweise oder gemäß Fig. 13b vollständig überdeckt.

Verwendbare Hydrogele sind beispielsweise in der DE 10 2007 034 580 A1 und der DE 10 2007 051 059 A1 beschrieben, so dass wegen weiterer Informationen auf diese Druckschriften verwiesen wird.

Die im Bereich der Dielektrophorese-Assemblierungsbereiche, also der Öffnungen oder Spalte 34 eingebrachten Hydrogele stabilisieren den Zellaufbau und können mit extrazellulären Matrixproteinen versetzt werden, wie es beispielsweise bei der Basalmembran in der Blut-Hirn-Schranke und der Blut-Luft-Schranke der Lunge der Fall ist.

Komplexe, asymmetrische Strukturen werden erzeugt, indem unterschiedliche Zelltypen auf verschiedenen Seiten des Hydrogels assembliert werden. Auf der einen Seite können z.B. Endothelzellen und auf der anderen Astrozyten etabliert werden.

Durch Einsatz eines auflösbaren Hydrogels lässt sich nach der Assemblierung ein direkter Kontakt zwischen den Zellen herstellen. Die Hydrogele weisen dazu beispielsweise Crosslinks auf, die durch Metalloproteasen geschnitten werden können. Es ist auch die Verwendung von Alginat-Gelen angedacht, die sich durch Zugabe von Ca²⁺ komplexierenden Substanzen wie EDT oder Citrat auflösen lassen.

Eine andere Möglichkeit, asymmetrische Strukturen aufzubauen, besteht darin, zwei getrennte Kanäle durch ein Mikrokapillararray zu verbinden, wie es prinzipiell bereits in der DE 10 2008 018 170 B4 beschrieben ist. Wird über ein derartiges Array von Mikroöffnungen ein dielektrophoretisches Feld angelegt, so werden jeweils nur durch einen der Kanäle eingespülte Zellen angesammelt, so dass ein asymmetrischer Zellaufbau realisiert werden kann.

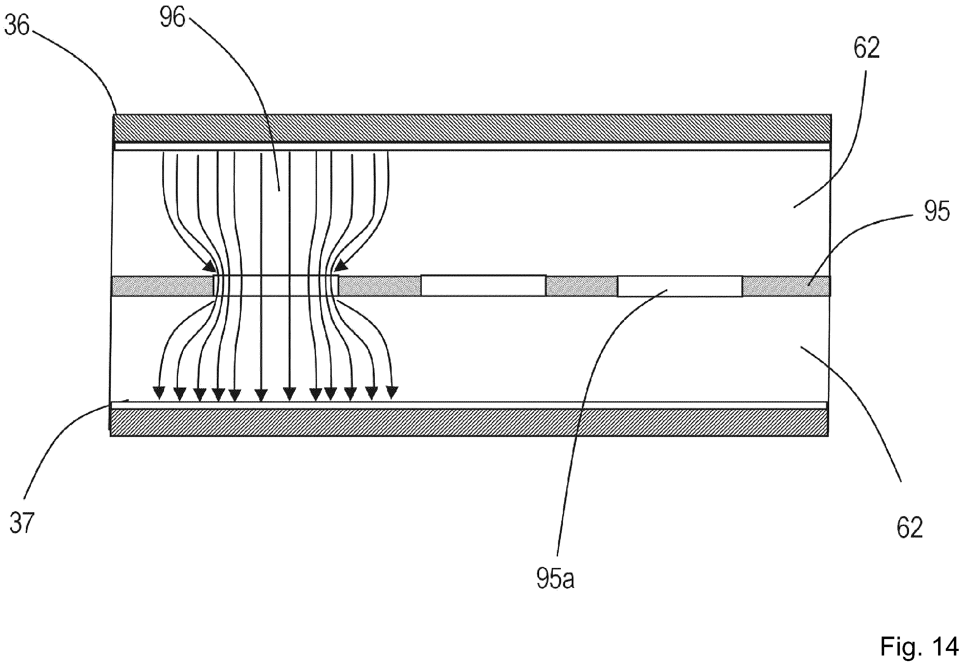

Statt eines derartigen Mikrokanalarrays kann auch eine isolierende Membran 95 mit Löchern 95a als Trennstruktur 94 zwischen den Mikrokanälen 38, 39 verwendet werden, wie es in Fig. 14 gezeigt ist. Dabei werden die Zellen durch das dielektrophoretische Feld in bzw. auf die Löcher 95a in der Membran gezogen, da diese Löcher 95a bei Anlegen einer Spannung ebenfalls lokale Feldinhomogenitäten 96 und damit dielektrophoretische Kräfte hervorrufen, wie es in Fig. 14 zu sehen ist, die ein Ausführungsbeispiel zur Ansammlung von komplexen Zellanordnungen in einem Probenanordnungsbereich 31 zeigt.

Für den Aufbau von Organen, die aus mehreren hintereinander geschalteten Unterstrukturen bestehen, wie es beispielsweise die Nierenkörperchen und der Nierentubulus bei der Niere darstellen, ist der aus der DE 10 2008 018 170 B4 bekannte Aufbau des Probenanordnungsbereiches nicht geeignet, wie die Erfinder inzwischen in eigenen Experimenten feststellen können.

Derartige Organstrukturen können erst durch die Reihenschaltung von zwei oder mehr Probenanordnungsbereichen realisiert werden, die einzeln und selektiv elektrisch adressierbar sind. Diese Probenanordnungsbereiche können entweder die gleichen oder verschiedene Mikrostrukturen für die Ausbildung des dielektrophoretischen Feldes aufweisen und Zellen in der für die jeweilige Unterstruktur geeigneten Anordnung positionieren.

Dabei werden zunächst Zellen für den ersten Probenanordnungsbereich 31 in den Probenchip 14 gespült und nur dieser erste Probenanordnungsbereich 31 mit einer Dielektrophoresespannung versehen, um die Zellen zu assemblieren. Nach gründlichem Spülen des Probenchips 14 werden dann die Zellen für den zweiten Probenanordnungsbereich 31 eingespült, das dielektrophoretische Feld nur an diesen zweiten Probenanordnungsbereich 31 angelegt und die zweite Unterstruktur assembliert.

Mit dem erfindungsgemäßen System lassen sich beispielsweise folgende Organ- und Gewebemodelle etablieren: Endothel-/Epithelschichten und Barrieren für die Blut-Hirn-Schranke, die Niere, den Darm und die Lunge; 3D-Gewebe von Tumoren, Metastasemodellen in Leber und Darm sowie Schilddrüse. Ferner kann die Ausdifferenzierung von Stammzellen etabliert und überwacht werden.

Wie bereits erwähnt sind die Kontrolleinheit 11 und die Anschlussplatte 12 wiederverwendbar, während die Probenchips 14 in der Regel Verbrauchsmaterialien sind.

Die Probenchips 14 können in einem Kit zusammen mit anderen Materialien bereitgestellt werden, um sie bereits für bestimmte Anwendungen vorzubereiten. Der Vorteil eines derartigen Kits besteht darin, dass alle benötigten Materialien zusammen mit dem mikrofluidischen Probenchip 14 als wirtschaftliche Einheit bereitgestellt werden, so dass die Einhaltung erforderlicher Standards und die Reinheit der Substanzen sichergestellt ist.

Zu den Bestandteilen des Kits zählen neben dem Probenchip 14 Medien, Reagenzien, Puffer und ggf. auch Zellen für folgende Anwendungen:

Bestimmung der Zellvitalität auf dem Probenchip, Extraktion von Nukleinsäure der kultivierten Zellen aus dem Probenchip, Kalibrierung der Zellkultur, Reinigung des Probenchips nach Beendigung des Versuches, Einbringen von Proteinbeschichtungen und/oder Hydrogelen in den Probenchip, Assemblierung von Zellen, Versand von vorkultivierten Probenchips.

In einer perfundierten 3D-Zellkultur können optische (beispielsweise Durchlicht, Fluoreszenz, Raman, SERS, FIB/SEM), elektrische (Impedanz, elektrochemische Sensoren) und biochemische (Analyse des Perfundats z.B. mittels HPLC/MS) Analyseverfahren durchgeführt werden.

### Beispiele für durchzuführende Assays

### a) Bestimmung von Nukleinsäuren in den Zellen

In diesem Assay müssen die Zellen lysiert und DNA/RNA aus dem Probenchip extrahiert werden, woraufhin die Nukleinsäuren dann über geeignete analytische Verfahren, wie beispielsweise Mikroarrays, Bead-Arrays, PCR- oder Sequenzierverfahren sowie massenspektrometrische Methoden weiter analysiert werden.

Ziel dieses Assays ist zum einen die Extraktion der DNA, um daran genetische Merkmale/Veränderungen der Zellen zu untersuchen, beispielsweise Mutationen in Tumorzellen oder Zelllinien. Zum anderen können über die Bestimmung der Menge und der Art der RNA in den Zellen Aussagen über die Hoch- oder Runterregulierung von Genen getroffen werden. Diese Assays dienen beispielsweise zur Untersuchung der Reaktion der Zellen auf bestimmte Substanzen.

Die Zellen werden dazu entweder chemisch über geeignete Substanzen oder elektrisch über im Probenchip integrierte Elektroden aufgeschlossen. Anschließend werden die Zellbestandteile aus dem Probenchip gespült und die Nukleinsäuren können über Standardverfahren vervielfältigt und/oder analysiert werden.

Der Probenchip bietet hier den Vorteil, dass nur sehr geringe Mengen an Reagenzien für den Zellaufschluss benötigt werden, oder dass sogar vollständig auf Reagenzien verzichtet werden kann, wenn die Zellen über das Anlegen einer niederfrequenten Spannung (< 100 kHz) an den bereits im Probenchip vorhandenen Elektroden lysiert werden.

Diese auf dem Probenchip realisierte Lyse bringt zahlreiche Vorteile: Dank des raschen Aufschlusses der Zellen ist der Abbau von Proteinen, DNA und RNA weitestgehend minimiert. Es kann vollständig auf chaotrope Reagenzien wie SDS oder Harnstoff verzichtet werden, die sich meist störend auf nachfolgende Assays auswirken. Zur Inaktivierung von Nukleasen und Proteasen können der Pufferlösung zudem Enzyminhibitoren beigefügt werden.

### b) Bestimmung von Enzymaktivitäten der Zellen

Hier ist beispielsweise an die Bestimmung von Metabolismusenzymaktivität in Zellen gedacht. Im Falle der Leber wären dies zum Beispiel Cytochrome P450 Enzyme (CYP), die hauptsächlich die Metabolisierung von Fremdstoffen, wie Medikamenten, im Körper katalysieren. Meist handelt es dabei um Oxidationsreaktionen bei denen Sauerstoffatome in die metabolisierte Substanz integriert werden.

Die Enzymaktivität in den Zellen wird dabei bestimmt, indem die Konzentration von Zellprodukten (z.B. Albumin bei Leberzellen) direkt im Perfundat bestimmt wird, oder die Umsetzung eines Enzymsubstrats durch die Zellen gemessen wird. Im zweiten Fall werden die Zellen mit Substraten der zu untersuchenden Enyzme inkubiert (z.B. Testosteron zur Bestimmung der CYP 3A4 Aktivität von Leberzellen), das Perfundat aufgesammelt und die Konzentration der Abbauprodukte im Perfundat bestimmt (z.B. 6β-Hydroxytestosteron welches von CYP3A4 aus Testosteron gebildet wird). Der Nachweis der Zellprodukte kann dabei z.B. über Immuno Assays (z.B. Enzyme-linked immunosorbent Assays zur Quantifizierung der Albuminkonzentration) oder mittels Chromatographie (z.B. High oder Ultra Performance Liquid Chromatography HPLC, UPLC) oder Massenspektroskopie erfolgen.

Ziel dieses Assays ist eine Überwachung der Stoffwechselfunktion von am Stoffwechsel beteiligten Zellen, wie beispielsweise der Leberzellen, um etwaige Änderungen als Reaktion auf zugeführte Substanzen feststellen zu können.

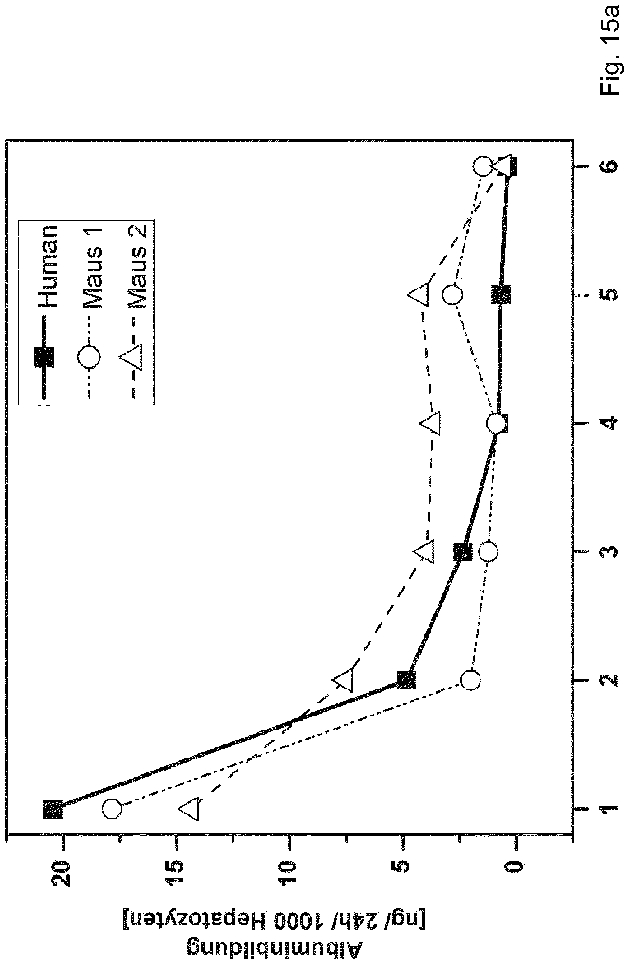

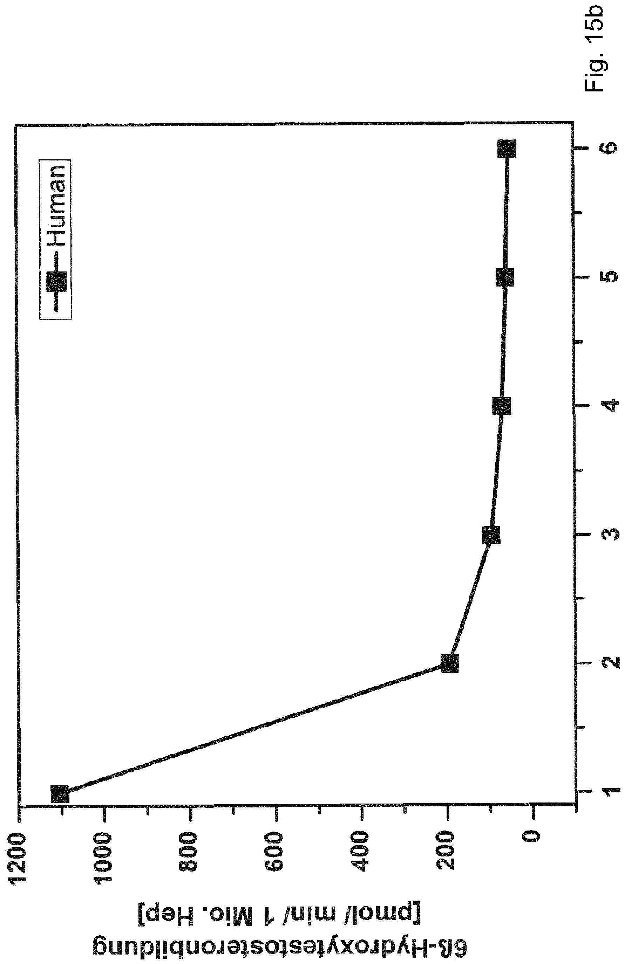

Fig. 15a und 15b zeigen als beispielhafte Anwendungen jeweils Ergebnisse von mit dem Probenchip durchgeführten Experimenten zum Rückgang der Albuminsyntheserate und der CYP3A4 Aktivität in einer organähnlichen Leberzellkultur, die mit hohen Wirkstoffkonzentrationen inkubiert wurden. Auf der Abszisse sind die Inkubationstage angegeben.

Fig. 15a zeigt Messungen der Albuminkonzentration aus 3 verschiedenen Experimenten mit humanen oder Maushepatozyten. Die Zellen wurden dabei dauerhaft mit 200 µM Phenacetin, 200 µM Testosteron und 100 µM 7-Hydroxycoumarin bei einer Flussrate von 3 µl/min inkubiert.

Fig. 15b zeigt Messungen der 6β-Hydroxytestosteron Konzentration in einem Experiment mit humanen Hepatozyten. Die Zellen wurden dabei dauerhaft mit 200 µM Phenacetin, 200 µM Testosteron und 100 µM 7-Hydroxycoumarin bei einer Flussrate von 3 µl/min inkubiert. Diese Messung gibt die Aktivität des Cytochrome P450 3A4 Enzyms wieder, welches die Umsetzung von Testosteron in 6β-Hydroxytestosteron katalysiert.

Bei dieser Art von Assay bietet der Probenchip den Vorteil, dass nur kleine Mengen an Reagenzien eingesetzt werden müssen. Außerdem wird die Konzentration an Substrat an den Zellen über die kontinuierliche Perfusion annähernd konstant gehalten, während die Stoffwechselprodukte ähnlich wie im Körper über den Medienfluss kontinuierlich abgeführt werden. So kann es nicht zu einer Sättigung an Produkt kommen, welches die Zellaktivität unnatürlich beeinflussen würde.

### c) Bestimmung der Toxizität von Substanzen

Bei diesem Assay geht es um die Bestimmung der Toxizität beispielsweise bei wiederholter Dosisgabe und die Bestimmung von Dosis-Zeitprofilen, wozu die Zellen mit Testsubstanzen inkubiert werden, die in definierten Zeitintervallen und Konzentrationen zugeführt werden. Dabei werden die Zellvitalität, die Zellaktivität und Veränderungen des Zellinhaltes - wie zum Beispiel Änderungen des Genoms oder Proteoms - über der Zeit bestimmt.

Ziel dieses Assays ist es, bei Toxizitätsuntersuchungen mit wiederholter Inkubation Langzeitwirkungen zu erkennen, die durch die wiederholte Einnahme von Substanzen entstehen können. Auf diese Weise können sowohl kurzfristige akute als auch durch längerfristige Inkubation ggf. auftretende chronisch toxische Effekte gemessen werden.

Die Zellen werden dazu über einen längeren Zeitraum abwechselnd mit den zu untersuchenden Substanzen (Induktionsphase) und reinem Zellkulturmedium (Erholungsphase) inkubiert, wobei die Reaktion der Zellen, insbesondere ihre Vitalität und Aktivität, überwacht wird. Dabei können sowohl die Konzentrationen als auch die Inkubationsdauer der Substanzen variiert werden.

Die Durchführung dieses Assays mit einem Probenchip bietet den Vorteil, dass wegen der Anwendung des perfundierten Probenchips kontinuierlich Substanzen in konstanten Konzentrationen oder mit definierten Konzentrationsprofilen appliziert werden können. Damit wird es erstmals möglich, die pharmakokinetischen Parameter eines Wirkstoffs in das Experiment einzubeziehen und so dessen Toxizität in einer experimentellen Situation zu untersuchen, die der *in-vivo* Situation nahe kommt.

Dank der Applikation von Substanzkonzentrationsgradienten über bestimmte Zeitspannen kann so die Medikamenteneinnahme und das Konzentrationsprofil in den Organen im Assay nachgebildet werden.

In der Perfusionseinheit können Konzentrationsprofile für die Wirkstoffe und sonstigen Substanzen programmiert und gesteuert werden. Ebenso können Inkubationsdauer, Flussgeschwindigkeit und Dauer der Erholungsphase über die Perfusionseinheit programmiert und kontrolliert eingehalten werden. Dieses Verfahren erlaubt es auch, Analyten im Perfusat zeitlich aufgelöst zu messen und mit dem Substanzkonzentrationsprofil zu korrelieren.

### d) Messung der Barrieregängigkeit von Substanzen

Um die qualitative und/oder quantitative Barrieregängigkeit von Substanzen zu messen, wird die Zellbarriere auf dem Probenchip etabliert und mit der jeweiligen Testsubstanz inkubiert, die Perfundate des Probenchips werden aufgesammelt und die Substanzkonzentration im Perfundat vor und hinter der Barriere bestimmt.

Im Körper gibt es verschiedene Barrieren, die die dahinterliegenden Regionen vor dem Eindringen unerwünschter Substanzen schützen; siehe beispielsweise die Blut-Hirn-Schranke, die Übergange Blut-Darm bzw. Blut-Harn, oder die Übergänge Luft-Blut in Nase, Bronchien und Lunge. Diese Situation wird im Probenchip nachempfunden.

Dabei kann zum einen überprüft werden, ob eine Substanz unbedenklich ist, weil sie nicht über die Membran tritt. Hier lässt sich beispielsweise das Eindringen oder Nicht-Eindringen von Umweltgiften in chemischer oder partikulärer Form (z.B. Nanopartikel) überprüfen. Ferner lassen sich neue Darreichungsformen für Wirkstoffe prüfen.

Zum anderen ist es für die Applikation von Medikamenten in bestimmte Körperbereiche wichtig, dass diese über die Barriere gehen, um ihren Wirkungsort zu erreichen. Dies gilt beispielsweise für das Übertreten der Blut-Hirn-Schranke zur Therapie von Gehirntumoren.

Im Probenchip werden dazu Zellbarrieren aufgebaut und mit Substanzen inkubiert. Zur Überprüfung der Barrieregängigkeit wird die Konzentration der Substanz vor und hinter der Barriere gemessen. Zudem kann getestet werden, ob bestimmte Stoffe die Barriere durchlässiger machen und sich damit eventuell schädlich auf einen Organismus auswirken.

Der Probenchip bietet gegenüber statischen Modellen den Vorteil, dass er die natürliche Flusssituation im Körper wiedergibt, wo Substanzen im Blutstrom an der Barriere vorbeiströmen und nicht - wie in einer statischen Zellkultur - in einer stehenden Flüssigkeit auf die Barriere wirken.

Durch die geringen Volumina im Probenchip sind außerdem nur geringe Mengen der oft nur begrenzt verfügbaren Substanzen nötig, um einen Test durchzuführen.

### e) Fixierung und Antikörperfärbung von Zellen

Auf dem Probenchip können für die Zwecke der Immunhistochemie Proteine mit Antikörpern und Färbereagenzien gezielt angefärbt werden.

Hierzu werden die betreffenden Zellen auf dem Probenchip fixiert, beispielsweise mit Alkoholen wie Methanol oder Ethanol, wobei das intrazelluläre Wasser aus den Zellen verdrängt und durch Alkohol ersetzt wird. Auf diese Weise sollen die Zellen haltbar gemacht werden.

Anschließend werden die Zellmembranen durch Zugabe von Detergenzien für Antikörper und andere Stoffe durchlässig gemacht. Die Antikörper/Färbereagenzien werden dann über die mikrofluidischen Kanäle 38 des Probenchips eingespült und so die Zellen mit diesem Medium inkubiert.

Durch die geringen Volumina des Probenchips werden nur geringe Mengen an oft teuren Antikörpern benötigt. Durch die kontinuierliche Perfusion gelangen mehr Antikörper in kurzer Zeit in Kontakt mit den Zellen, wodurch sich die Dauer der Färbung gegenüber statischen Zellkulturen verkürzt.

### f) Fixierung und Einbettung von Zellen

Auf dem Probenchip können auch Zellen für Untersuchungen mittels invasiver Verfahren, beispielsweise für die Elektronenmikroskopie, präpariert werden.

Alle Reagenzien werden über die mikrofluidischen Kanäle des Probenchips zugeführt. Zunächst werden die Zellen im Probenchip beispielsweise mit Methanol fixiert. Zur Vorbereitung auf die Elektronenmikroskopie werden die Zellen dann getrocknet, indem beispielsweise das Wasser durch Ethanol ersetzt wird, und mit einem Kontrastmittel wie beispielsweise Osmiumtetroxid inkubiert. Anschließend werden die Zellen in eine Matrix eingebettet. Diese unterscheidet sich je nach anschließender Untersuchungsmethode, für Gewebeschnitte wird beispielsweise Paraffin und für die Elektronenmikroskopie Epoxidharz verwendet. Dieses Einbetten geschieht dadurch, dass Paraffin oder Epoxidharz durch die mikrofluidischen Kanäle zu den Zellen gespült wird. Nach diesem Einbetten wird die Abdeckung des Probenchips entfernt, die in die Matrix eingebetteten Zellen entnommen, geschnitten und die Schnitte entsprechend untersucht.

### g) Kalibrierung des Probenchips

Bevor Zellfunktionsassays wie Toxizität, Vitalität oder Enzymaktivität durchgeführt werden können, kann die Ausgangsfunktionalität der Zellen festgestellt werden.

Dazu wird eine der Probenkanaleinheiten 15 auf dem Probenchip 14 als Referenzkanal verwendet. Hier wird die Funktion der Zellen ohne den Einfluss der Substanzen überwacht. Dafür werden festgelegte Parameter vor der Inbetriebnahme der anderen Probenkanaleinheiten 15 überprüft. Zusätzlich zur Überprüfung der Zellvitalität richten sich diese Parameter nach der Art der Organkultur, bei einer Leberkultur wird beispielsweise die Albuminproduktion, bei der Blut-Hirn-Schranke die elektrische Impedanz der Zellbarriere überwacht.

Wie sich aus der vorstehenden Beschreibung der unterschiedlichen Assays ergibt, die mit dem Probenchip bzw. dem neuen System durchgeführt werden können, eröffnet das neue System viele neue Anwendungsmöglichkeiten.

### Anwendungsbeispiele für den Probenchip

### 1) Toxitätsmechanismen

Bei dieser Anwendung soll die Ursache für die toxische Wirkung von Substanzen bestimmt werden. Die Zellkultur wird dazu mit toxischen Substanzen inkubiert und die Wirkung der Substanz auf verschiedene Zellfunktionen untersucht. Hierzu zählt beispielsweise die Inhibition von Enzymen, das Hervorrufen von Entzündungsreaktionen oder die Zerstörung von Zell-Zellinteraktionen.

Durch die auf dem Probenchip mögliche Etablierung einer Zellkultur, die der *in vivo*-Kultursituation sehr ähnlich ist, ist es damit erstmals möglich, den Mechanismen von Toxizität in einem human ähnlichen Modell zu bestimmen.

### 2) Metastasenbildung: Einwandern von Tumorzellen in "Mikrogewebe"

Das neue System ermöglicht auch die Etablierung von Modellen zur Untersuchung von Tumorbildung. Nach Aufbau einer Organkultur aus gesunden Zellen werden über die mikrofluidischen Kanäle Tumorzellen, z.B. zirkulierende Tumorzellen, eingespült. Über mikroskopische Überwachung wird das Verhalten der Tumorzellen in der Gewebekultur beobachtet. Zum Beispiel kann das Eindringen in das Zellaggregat, die Verdrängung von Zellen oder das Wachstum des Tumors optisch überwacht werden.

Durch die im Probenanordnungsbereich vorhandenen Elektroden kann durch Dielektrophorese das Eindringen der Tumorzellen in die Organkultur, also die Metastasierung gefördert werden.

Gleichzeitig kann die Funktionalität der Zellen überwacht werden, indem der oben unter b) beschriebene Assay verwendet wird.

Anschließend können sowohl der Tumor als auch die gesunden Zellen lysiert und ihre Bestandteile, also im Wesentlichen die Proteine und die Nukleinsäuren, analysiert werden; siehe oben Assay a).

Diese kontinuierliche Untersuchung der Tumorentstehung an derselben Organkultur ist erstmals mit dem neuen System und dem Probenchip möglich. Bisher verwendete Zellkulturen waren für diese Art der Untersuchung nicht körperähnlich genug. In Tiermodellen ist es darüber hinaus nahezu unmöglich, die Entstehung von Tumoren schon in der Anfangsphase zu untersuchen oder gar kontinuierlich zu verfolgen, da eine Beobachtung am lebenden Tier nicht möglich ist und daher die Tumorgenese nur zu verschiedenen diskreten Zeitpunkten - ggf. sogar nur in verschiedenen Tieren - beobachtet werden kann.

### 3) Wirkstofftestung an onkologischem Material/Tumormodell

Mit dem neuen System ist es auch möglich, die Wirkung von Substanzen auf Tumorzellen zu untersuchen. Dazu werden Zellen - je nach Art des untersuchten Tumors - in einem tumorähnlichen Aufbau im Probenchip assembliert und kultiviert. Die Zellen werden dann mit Wirkstoffkandidaten inkubiert und ihre Reaktion auf die Wirkstoffe überwacht. Hier werden insbesondere die Vitalität und Funktionalität der Tumorzellen sowie das Schrumpfen/Wachsen des Tumors beobachtet.

Durch die Kultivierung der Zellen als Mono- oder Kokultur in einer *in vivo*-ähnlichen Umgebung ist die Reaktion der Zellen auf die Wirkstoffkandidaten der realen Situation ähnlicher als dies in bisherigen Modellen der Fall war.

### 4) Pathogeninteraktionen mit Gewebe

Ziel dieser Anwendung ist es, das Eindringen von Pathogenen, also beispielsweise Bakterien, Viren oder Parasiten, in Gewebe zu untersuchen.

Dazu wird zunächst eine Organ-/Gewebekultur im Probenchip etabliert. Anschließend werden die Pathogene durch die mikrofluidischen Kanäle eingespült und ihr Verhalten wird in der Zellkultur optisch überwacht. Beispielsweise kann das Eindringen in das aufgebaute Gewebe beobachtet werden. Zusätzlich wird untersucht, ob die Pathogene die Funktionalität der Zellen in Kultur beeinflussen. Hier werden beispielsweise die Vitalität und die Enzymaktivität überprüft.

Im Probenchip kann das Eindringen von Krankheitserregern in ein Gewebe/ein Organ kontinuierlich beobachtet werden. Durch Kultivierung der Zellen in einer *in vivo*-ähnlichen Umgebung ist die Reaktion der Zellen auf die Pathogene der realen Situation ähnlicher, als dies in bisherigen Modellen der Fall war.

### 5) Gegenseitige Beeinflussung von Substanzen in ihrer Wirkung

Bei dieser Anwendung wird untersucht, wie sich die toxische Wirkung bei paralleler Verabreichung mehrerer Wirkstoffe (Drug-Drug-Interaction) verändert. Dazu wird die Zellkultur mit zwei oder mehreren Wirkstoffen entweder gleichzeitig oder sequenziell inkubiert und die Vitalität der Zellen überwacht.

In Tierversuchen gewonnene Erkenntnisse über Medikamentenwechselwirkungen lassen sich nicht ohne weiteres auf den Menschen übertragen, da Substanzen im Tier oft mit anderer Geschwindigkeit abgebaut werden. So kann es sein, dass im Menschen ein Wirkstoff A schneller als ein Wirkstoff B abgebaut wird, während es im Tier genau umgekehrt abläuft. Daher sind Tierversuche zur Abschätzung von Drug-Drug-Interaktionen bei humanen Patienten ungeeignet.

Im Probenchip lassen sich diese Wechselwirkungen nun an humanen Zellen und organähnlichen Zellverbänden testen. Diese Situation ist der humanen Situation ähnlicher als bisherige Anwendungen, so dass eine bessere Übertragbarkeit auf die humane Situation möglich ist, als bei einem Tierversuch oder in bisher verwendeten Zelltests.

### 6) Mechanismen für die Barrieregängigkeit von Substanzen

Bei dieser Anwendung soll die Toxizität von Substanzen anhand des Durchtrittsverhaltens von Wirkstoffen/Substanzen über Barrieren (Blut-Hirn-Schranke, Darm, Niere) untersucht werden.

Der Ablauf erfolgt wie oben in den Assays c) - e), wobei sich die entsprechenden Vorteile ergeben.

Weitere Beispiele sind nachfolgend skizzenhaft in Form von Klauseln im Sinne der Entscheidung J 15/88 der Beschwerdekammer des europäischen Patentamts wiedergegeben:
Klausel 1. Anschlussplatte für ein System zur Durchführung von Untersuchungen an und/oder mit biologischem Probenmaterial (35), die eine mechanische Aufnahmevorrichtung (13) für einen mikrofluidischen Probenchip (14), in dem das zu untersuchende biologische Probenmaterial (35) angeordnet und ggf. kultiviert wird, äußere Anschlusselemente (23), um die Anschlussplatte (12) zur Übertragung von Medien und/oder Signalen lösbar mit einer Kontrolleinheit (11) zu verbinden, und innere Anschlusselemente (21) aufweist, um die Anschlussplatte (12) zur Übertragung von Medien und/oder Signalen lösbar mit dem Probenchip (14) zu verbinden.
Klausel 2. Anschlussplatte nach Klausel 1, dadurch gekennzeichnet, dass sie zumindest ein Funktionselement (25) aufweist, das zwischen den äußeren und den inneren Anschlusselementen (23, 21) angeordnet ist.
Klausel 3. Anschlussplatte nach Klausel 1 oder 2, dadurch gekennzeichnet, dass das zumindest eine Funktionselemente (25) dazu ausgelegt ist, zumindest eine Aufgabe zu erfüllen, die ausgewählt ist aus der Gruppe, die die elektrische und optische Aufbereitung, Verteilung und Zusammenführung sowie Zwischenspeicherung von Steuer- und Messsignalen, die Speicherung, den Transport und die Verteilung von Flüssigkeiten und Gasen, das Abtrennen von Gasbestandteilen aus den Flüssigkeiten in aktiven oder passiven Blasenfallen, die Erzeugung elektrischer Pulse, die Erzeugung elektrischer und/oder magnetischer Gleich- und/oder Wechselfelder, sowie die Übertragung von Drucksignalen umfasst.
Klausel 4. Anschlussplatte nach einer der Klauseln 1 bis 3, dadurch gekennzeichnet, dass das zumindest eine Funktionselement (25) als optisch empfindlicher, elektrischer, chemischer oder biochemischer Sensor ausgelegt ist.
Klausel 5. Anschlussplatte nach einer der Klauseln 1 bis 4, dadurch gekennzeichnet, dass die inneren Anschlusselemente (21) zum blasenfreien Ankoppeln an den Probenchip (14) und die äußeren Anschlusselemente (23) zum blasenfreien Ankoppeln an die Kontrolleinheit (11) ausgebildet sind.
Klausel 6. Anschlussplatte nach einer der Klauseln 1 bis 5, dadurch gekennzeichnet, dass die Aufnahmevorrichtung (13) Spannelemente (65, 66) zum mechanischen Halten des Probenchips (14) aufweist.
Klausel 7. Anschlussplatte nach Klausel 6, dadurch gekennzeichnet, dass in der Aufnahmevorrichtung (13) eine optisch dünne Abdeckung (64) vorgesehen ist.
Klausel 8. Mikrofluidischer Probenchip, insbesondere für eine Anschlussplatte (12) nach einer der Klauseln 1 bis 7, mit zumindest einer Probenkanaleinheit (15), in der ein mikrofluidischer Probenanordnungsbereich (31) vorgesehen ist, in dem biologisches Probenmaterial (35) angeordnet und ggf. kultiviert wird, wobei die Probenkanaleinheit (15) mit einem Anschlussbereich (16) zur Übertragung von Medien und/oder Signalen versehen ist.
Klausel 9. Probenchip nach Klausel 8, dadurch gekennzeichnet, dass das zwischen dem Probenanordnungsbereich (31) und dem Anschlussbereich (16) zumindest ein Funktionselement (41, 42, 45, 48, 49, 53, 54, 55) angeordnet ist.
Klausel 10. Probenchip nach Klausel 8 oder 9, dadurch gekennzeichnet, dass das zumindest eine Funktionselement (41, 42, 45, 48, 49, 53, 54, 55) als optisch empfindlicher, elektrischer, chemischer oder biochemischer Sensor ausgelegt ist.
Klausel 11. Probenchip nach Klausel 8 oder 9, dadurch gekennzeichnet, dass das zumindest eine Funktionselemente (41, 42, 45, 48, 49, 53, 54, 55) dazu ausgelegt ist, zumindest eine Aufgabe zu erfüllen, die ausgewählt ist aus der Gruppe, die die elektrische und optische, Aufbereitung, Verteilung und Zusammenführung sowie Zwischenspeicherung von Steuer- und Messsignalen, die Speicherung, den Transport und die Verteilung von Flüssigkeiten und Gasen, das Abtrennen von Gasbestandteilen aus den Flüssigkeiten in aktiven oder passiven Blasenfallen, die Erzeugung elektrischer Pulse, die Erzeugung elektrischer und/oder magnetischer Gleich- und/oder Wechselfelder, sowie die Übertragung von Drucksignalen umfasst.
Klausel 12. Probenchip nach einer der Klauseln 8 bis 11, dadurch gekennzeichnet, dass in dem Probenanordnungsbereich (31) zumindest zwei Elektroden (36, 37) zur Erzeugung eines elektrischen Feldes vorgesehen sind.
Klausel 13. Probenchip nach Klausel 12, dadurch gekennzeichnet, dass das die Elektroden (36, 37) zur Impedanzmessung ausgelegt sind.
Klausel 14. Probenchip nach Klausel 12 oder 13, dadurch gekennzeichnet, dass das die Elektroden (36, 37) zur Erzeugung elektrischer Pulse ausgelegt sind.
Klausel 15. Probenchip nach einer der Klauseln 12 bis 14, dadurch gekennzeichnet, dass das die Elektroden (36, 37) als elektrisch leitende Polymerelektroden ausgebildet sind.
Klausel 16. Probenchip nach einer der Klauseln 8 bis 15, dadurch gekennzeichnet, dass in dem Probenanordnungsbereich (31) zumindest ein von zwei Stegen (32, 33) begrenzter Spalt (34) vorgesehen ist, der zwei mikrofluidische Kanäle (62) miteinander verbindet und in dem eine Trennstruktur (94, 95, 97) angeordnet ist.
Klausel 17. Probenchip nach Klausel 16, dadurch gekennzeichnet, dass die Trennstruktur (94, 95, 97) eine Membran (95) mit Löchern (95a) umfasst.
Klausel 18. Probenchip nach Klausel 16, dadurch gekennzeichnet, dass die Trennstruktur (94, 95, 97) ein Hydrogel (97) umfasst.
Klausel 19. Probenchip nach einer der Klauseln 8 bis 18, dadurch gekennzeichnet, dass er ein Trägersubstrat (14a) umfasst, in dem die zumindest eine Probenkanaleinheit (15) ausgebildet ist, und das mit einem Deckel (14b) verschlossen ist, der vorzugsweise zumindest bereichsweise optisch dünn ausgelegt ist.
Klausel 20. Probenchip nach einer der Klauseln 8 bis 19, dadurch gekennzeichnet, dass er zumindest eine passive Blasenfalle (45) mit einer Membran (87) umfasst.
Klausel 21. System zur Durchführung von Untersuchungen an und/oder mit in einem Probenanordnungsbereich (31) eines mikrofluidischen Probenchips (14) nach einer der Klauseln 8 bis 20 befindlichen biologischen Probenmaterial, mit einer Anschlussplatte (12) nach einer der Klauseln 1 bis 7 zur Aufnahme des Probenchips (14), und zumindest einer Kontrolleinheit (11), mit der die Anschlussplatte (12) zur Übertragung von Medien und/oder Signalen lösbar verbindbar ist.
Klausel 22. System nach Klausel 21, dadurch gekennzeichnet, dass die Kontrolleinheit (11) als Dielektrophoreseeinheit zum Primen des Probenchips (14) und zum Ansammeln des biologischen Probenmaterials (35) in dem Probenanordnungsbereich (31) ausgebildet ist.
Klausel 23. System nach Klausel 21 oder Klausel 22, dadurch gekennzeichnet, dass die Kontrolleinheit als Perfusionseinheit zur Versorgung des Probenmaterials (35) mit Medium und zur Inkubation des Probenmaterials (35) über einen Messzeitraum sowie zur Erfassung von elektrischen Messwerten und/oder zur Entnahme für flüssige Messproben ausgebildet ist.
Klausel 24. Verfahren zur Durchführung von Untersuchung an und/oder mit biologischem Probenmaterial (35) in einem System, das zumindest einen mikrofluidischen Probenanordnungsbereich aufweist, in dem biologisches Probenmaterial (35) angeordnet und ggf. kultiviert wird, mit den Schritten: a) das biologische Probenmaterial (35) wird in dem Probenanordnungsbereich (31) assembliert, b) das Probenmaterial (35) wird mit zumindest einer Substanz inkubiert, die über mikrofluidische Kanäle (38) zugeführt wird, und c) das Probenmaterial (35) wird untersucht.
Klausel 25. Verfahren nach Klausel 24, bei dem in Schritt a) zumindest ein organähnliches Zellkulturmodell in dem Probenanordnungsbereich (31) etabliert wird.
Klausel 26. Verfahren nach Klausel 24 oder 25, bei dem in Schritt c) mit zumindest einem Analyseverfahren eine Eigenschaft oder Eigenschaftsänderung des Probenmaterials untersucht wird, die ausgewählt ist aus Metabolom, Enzymaktivität, Proteom, Genom, Genexpression, Sekretion von Markern, morphologische Erscheinung, Zellvitalität, Zellorganellenfunktion, Interaktion zwischen Zellen, Interaktion zwischen Zellen und Umgebung im Probenanordnungsbereich, Proliferation.
Klausel 27. Verfahren nach einer der Klauseln 24 bis 26, bei dem in Schritt c) (die) Zellen elektrisch über in dem Probenanordnungsbereich integrierte Elektroden aufgeschlossen werden.
28. Verfahren nach Klausel 26 oder 27, bei dem das Analyseverfahren ausgewählt ist aus Immunhistochemie, Mikroskopie, Elektronenmikroskopie, Anfärbung von Zellbestandteilen, Analyse von gelösten Bestandteilen mittels Trennverfahren (Chromatographie, Elektrophorese, Massenspektrometrie), Colorimetrie, Immunoassays, Nukleinsäure-Analytik, PCR (Polymerase Chain Reaction), FISH (Fluorescence in situ hybridization), DNA-Sequenzierung, Spektroskopieverfahren (Infrarotspektroskopie, Ramanspektroskopie, NMR-Spektroskopie, Fluoreszenz-, UV/VIS-Spektroskopie).
Klausel 29. Verfahren nach einer der Klauseln 24 bis 28, bei dem in Schritt c) die qualitative und/oder quantitative Wirkung der zugeführten zumindest einen Substanz auf das Probenmaterial untersucht wird.
Klausel 30. Verfahren nach einer der Klauseln 24 bis 29, bei dem eine vergleichende Untersuchung an einem Probenmaterial, das mit der Substanz inkubiert wird, und einem Probenmaterial, das nicht oder mit einer anderen Substanz inkubiert wird, durchgeführt wird.
Klausel 31. Verfahren nach einer der Klauseln 24 bis 30, bei dem in Schritt a) ein leberähnliches Zellkulturmodell assembliert und kultiviert wird, das in Schritt b) mit zumindest einer potentiell toxischen Substanz inkubiert wird, wobei in Schritt c) die Wirkung der Substanz auf zumindest eine Zellfunktion untersucht wird.
Klausel 32. Verfahren nach einer der Klauseln 24 bis 30, bei dem in Schritt a) Tumorzellen in einer organähnlichen Zellkultur assembliert und kultiviert werden, die in Schritt b) mit zumindest einem onkologischen Wirkstoff inkubiert wird, wobei in Schritt c) eine Veränderung der Zellkultur untersucht wird.
Klausel 33. Verfahren nach einer der Klauseln 24 bis 30, bei dem in Schritt a) eine biologische Zellbarriere in einer organähnlichen Zellkultur assembliert und kultiviert wird, die in Schritt b) auf einer Seite der Zellbarriere mit zumindest einem Wirkstoff inkubiert wird, wobei in Schritt c) die Konzentration des Wirkstoffes auf der anderen Seite der Zellkultur untersucht wird.
Klausel 34. Verfahren nach einer der Klauseln 24 bis 30, bei dem in Schritt a) biologische Zellen in einer organähnlichen Zellkultur assembliert und kultiviert werden, die in Schritt b) mit zumindest einem Pathogen inkubiert wird, wobei in Schritt c) die Reaktion der Zellkultur auf die Inkubation mit dem zumindest einen Pathogen untersucht wird.
Klausel 35. Verfahren nach einer der Klauseln 24 bis 30, bei dem in Schritt a) biologische Zellen in einer organähnlichen Zellkultur assembliert und fixiert werden, an die in Schritt b) zumindest ein Färbemittel gebunden wird, wobei in Schritt c) die Färbung mittels optischer Verfahren untersucht wird.
Klausel 36. Verfahren nach einer der Klauseln 24 bis 30, bei dem in Schritt a) biologische Zellen in einer organähnlichen Zellkultur assembliert und fixiert werden, die in Schritt b) getrocknet sowie mit einem Kontrastmittel inkubiert und dann in eine Matrix eingebettet werden, wobei in Schritt c) die in die Matrix eingebetteten Zellen entnommen und untersucht werden.
Klausel 37. Verfahren nach einer der Klauseln 24 bis 30, bei dem in Schritt a) biologische Zellen in einer organähnlichen Zellkultur assembliert und kultiviert werden, die in Schritt b) mit Tumorzellen inkubiert wird, wobei in Schritt c) das Verhalten der Tumorzellen und der Zellkultur untersucht wird.
Klausel 38 Verfahren nach Klausel 37, bei dem in Schritt b) die Interaktion der Tumorzellen mit der Zellkultur durch Dielektrophorese gefördert wird.
Klausel 39. Verfahren nach einer der Klauseln 24 bis 30, bei dem in Schritt a) biologische Zellen in einer organähnlichen Zellkultur assembliert und kultiviert werden, die in Schritt b) mit zumindest zwei Wirkstoffen inkubiert wird, wobei in Schritt c) das Verhalten der Zellkultur untersucht wird.
40. Verfahren nach einer der Klauseln 24 bis 30, bei dem in Schritt a) in einem ersten Probenanordnungsbereich biologische Zellen in einer ersten organähnlichen Zellkultur und in einem zweiten Probenanordnungsbereich biologische Zellen in einer zweiten organähnlichen Zellkultur assembliert und kultiviert werden.
Klausel 41. Verfahren nach Klausel 40, bei dem der erste und der zweite Probenanordnungsbereich fluidisch in Reihe geschaltet sind, wobei in Schritt b) die erste Zellkultur mit zumindest einem Wirkstoff perfundiert wird, und die zweite Zellkultur mit dem Effluent der ersten Zellkultur perfundiert wird, und wobei in Schritt c) die Reaktion der zweiten Zellkultur auf die Perfusion mit dem Effluent untersucht wird.
Klausel 42. Verfahren nach einer der Klauseln 24 bis 41, bei dem das System zumindest einen Probenchip nach einer der Klauseln 8 bis 20 umfasst.
Klausel 43. Verfahren nach einer der Klauseln 24 bis 42, bei dem das System zumindest eine Anschlussplatte nach einer der Klauseln 1 bis 7 umfasst.
Klausel 44. Verfahren nach einer der Klauseln 24 bis 43, bei dem das System das System nach einer der Klauseln 21 bis 23 ist.

## Patentansprüche

1. Verfahren zur Durchführung von Untersuchung an und/oder mit biologischem Probenmaterial (35) in einem System, das zumindest einen mikrofluidischen Probenanordnungsbereich aufweist, in dem biologisches Probenmaterial (35) angeordnet und ggf. kultiviert wird, mit den Schritten:
a) das biologische Probenmaterial (35) wird in dem Probenanordnungsbereich (31) assembliert,
b) das Probenmaterial (35) wird mit zumindest einer Substanz inkubiert, die über mikrofluidische Kanäle (38) zugeführt wird, und
c) das Probenmaterial (35) wird untersucht.

2. Verfahren nach Anspruch 1, bei dem in Schritt a) zumindest ein organähnliches Zellkulturmodell in dem Probenanordnungsbereich (31) etabliert wird.

3. Verfahren nach einem der vorhergehenden Ansprüche, bei dem in Schritt c) mit zumindest einem Analyseverfahren eine Eigenschaft oder Eigenschaftsänderung des Probenmaterials untersucht wird, die ausgewählt ist aus Metabolom, Enzymaktivität, Proteom, Genom, Genexpression, Sekretion von Markern, morphologische Erscheinung, Zellvitalität, Zellorganellenfunktion, Interaktion zwischen Zellen, Interaktion zwischen Zellen und Umgebung im Probenanordnungsbereich, Proliferation; insbesondere wobei das Analyseverfahren ausgewählt ist aus Immunhistochemie, Mikroskopie, Elektronenmikroskopie, Anfärbung von Zellbestandteilen, Analyse von gelösten Bestandteilen mittels Trennverfahren (Chromatographie, Elektrophorese, Massenspektrometrie), Colorimetrie, Immunoassays, Nukleinsäure-Analytik, PCR (Polymerase Chain Reaction), FISH (Fluorescence in situ hybridization), DNA-Sequenzierung, Spektroskopieverfahren (Infrarotspektroskopie, Ramanspektroskopie, NMR-Spektroskopie, Fluoreszenz-, UV/VIS-Spektroskopie).

4. Verfahren nach einem der vorhergehenden Ansprüche , bei dem in Schritt c) Zellen elektrisch über in dem Probenanordnungsbereich integrierte Elektroden aufgeschlossen werden.

5. Verfahren nach einem der vorhergehenden Ansprüche , bei dem in Schritt c) die qualitative und/oder quantitative Wirkung der zugeführten zumindest einen Substanz auf das Probenmaterial untersucht wird.

6. Verfahren nach einem der Ansprüche 1 bis 5, bei dem in Schritt a) ein leberähnliches Zellkulturmodell assembliert und kultiviert wird, das in Schritt b) mit zumindest einer potentiell toxischen Substanz inkubiert wird, wobei in Schritt c) die Wirkung der Substanz auf zumindest eine Zellfunktion untersucht wird.

7. Verfahren nach einem der Ansprüche 1 bis 5, bei dem in Schritt a) Tumorzellen in einer organähnlichen Zellkultur assembliert und kultiviert werden, die in Schritt b) mit zumindest einem onkologischen Wirkstoff inkubiert wird, wobei in Schritt c) eine Veränderung der Zellkultur untersucht wird.

8. Verfahren nach einem der Ansprüche 1 bis 5, bei dem in Schritt a) eine biologische Zellbarriere in einer organähnlichen Zellkultur assembliert und kultiviert wird, die in Schritt b) auf einer Seite der Zellbarriere mit zumindest einem Wirkstoff inkubiert wird, wobei in Schritt c) die Konzentration des Wirkstoffes auf der anderen Seite der Zellkultur untersucht wird.

9. Verfahren nach einem der Ansprüche 1 bis 5, bei dem in Schritt a) biologische Zellen in einer organähnlichen Zellkultur assembliert und kultiviert werden, die in Schritt b) mit zumindest einem Pathogen inkubiert wird, wobei in Schritt c) die Reaktion der Zellkultur auf die Inkubation mit dem zumindest einen Pathogen untersucht wird.

10. Verfahren nach einem der Ansprüche 1 bis 5, bei dem in Schritt a) biologische Zellen in einer organähnlichen Zellkultur assembliert und fixiert werden, an die in Schritt b) zumindest ein Färbemittel gebunden wird, wobei in Schritt c) die Färbung mittels optischer Verfahren untersucht wird; oder bei dem in Schritt a) biologische Zellen in einer organähnlichen Zellkultur assembliert und fixiert werden, die in Schritt b) getrocknet sowie mit einem Kontrastmittel inkubiert und dann in eine Matrix eingebettet werden, wobei in Schritt c) die in die Matrix eingebetteten Zellen entnommen und untersucht werden.

11. Verfahren nach einem der Ansprüche 1 bis 5, bei dem in Schritt a) biologische Zellen in einer organähnlichen Zellkultur assembliert und kultiviert werden, die in Schritt b) mit Tumorzellen inkubiert wird, wobei in Schritt c) das Verhalten der Tumorzellen und der Zellkultur untersucht wird.

12. Verfahren nach Anspruch 11, bei dem in Schritt b) die Interaktion der Tumorzellen mit der Zellkultur durch Dielektrophorese gefördert wird.

13. Verfahren nach einem der Ansprüche 1 bis 5, bei dem in Schritt a) biologische Zellen in einer organähnlichen Zellkultur assembliert und kultiviert werden, die in Schritt b) mit zumindest zwei Wirkstoffen inkubiert wird, wobei in Schritt c) das Verhalten der Zellkultur untersucht wird.

14. Verfahren nach einem der Ansprüche 1 bis 5, bei dem in Schritt a) in einem ersten Probenanordnungsbereich biologische Zellen in einer ersten organähnlichen Zellkultur und in einem zweiten Probenanordnungsbereich biologische Zellen in einer zweiten organähnlichen Zellkultur assembliert und kultiviert werden; insbesondere wobei der erste und der zweite Probenanordnungsbereich fluidisch in Reihe geschaltet sind, wobei in Schritt b) die erste Zellkultur mit zumindest einem Wirkstoff perfundiert wird, und die zweite Zellkultur mit dem Effluent der ersten Zellkultur perfundiert wird, und wobei in Schritt c) die Reaktion der zweiten Zellkultur auf die Perfusion mit dem Effluent untersucht wird.

15. Verfahren nach einem der vorhergehenden Ansprüche , bei dem das System zumindest einen Probenchip und/ oder eine Anschlussplatte umfasst.
